# EUROPÄISCHE PATENTSCHRIFT

(11) **EP 2 051 992 B1**
(45) Veröffentlichungstag und Bekanntmachung des Hinweises auf die Patenterteilung: **04.08.2010**
(21) Anmeldenummer: 07786713.3
(22) Anmeldetag: 24.08.2007
(51) Int. Cl.: C07K 7/06, A61K 38/08

(54) **VERFAHREN ZUR HERSTELLUNG VON KONDENSATIONSPRODUKTEN AUS N-SUBSTITUIERTEN GLYCINDERIVATEN (PEPTOIDE) ÜBER SEQUENTIELLE UGI-MEHRKOMPONENTENREAKTIONEN**
METHOD FOR PRODUCING CONDENSATION PRODUCTS FROM N-SUBSTITUTED GLYCINE DERIVATIVES (PEPTOIDS) BY SEQUENTIAL UGI-MULTICOMPONENT REACTIONS
PROCÉDÉ DE FABRICATION DE PRODUITS DE CONDENSATION DE DÉRIVÉS DE GLYCINE N-SUBSTITUÉE (PEPTOÏDES) PAR DES RÉACTIONS SÉQUENTIELLES DE UGI À PLUSIEURS COMPOSANTS

(30) Priorität: 24.08.2006 DE 102006039615
(43) Veröffentlichungstag der Anmeldung: 29.04.2009
(73) Patentinhaber: Leibniz-Institut für Pflanzenbiochemie (IPB), 06120 Halle (Saale) (DE)
(72) Erfinder: WESSJOHANN, Ludger, A., 06120 Halle (Saale) (DE); TRAN THI PHUONG, Thao, Ha Noi (VN); WESTERMANN, Bernhard, 06120 Halle (Saale) (DE)
(74) Vertreter: Müller-Boré & Partner Patentanwälte
(86) Internationale Anmeldenummer: PCT/EP2007/007458
(87) Internationale Veröffentlichungsnummer: WO 2008/022800

(56) Entgegenhaltungen:
- WO-A-01/88178
- WO-A-91/19735
- WO-A-98/09641
- WO-A-98/51697
- WO-A-99/26968
- US-A1- 2002 019 013
- US-A1- 2002 115 612
- MASIP I ET AL.: "Peptoids As Source of Compounds Eliciting Antibacterial Activity" COMBINATORIAL CHEMISTRY & HIGH THROUGHPUT SCREENING, Bd. 8, Nr. 3, Mai 2005 (2005-05), Seiten 235-239, XP008086295 ISSN: 1386-2073
- JIN M K & ROY R: "New prototypical O-linked-glycopeptidomimetics corresponding to the linkage region of proteoglycans" CARBOHYDRATE RESEARCH, ELSEVIER SCIENTIFIC PUBLISHING COMPANY. AMSTERDAM, NL, Bd. 298, Nr. 3, 5. März 1997 (1997-03-05), Seiten 173-179, XP004091179 ISSN: 0008-6215
- TANG Y-C & DEBER CM: "Hydrophobicity and Helicity of Membrane-Interactive Peptides Containing Peptoid Residues" BIOPOLYMERS, Bd. 65, 2002, Seiten 254-262, XP002460493

## Beschreibung

Die vorliegende Erfindung betrifft ein Verfahren zur Herstellung von Kondensationsprodukten (Peptoiden) aus N-substituierten Glycinderivaten über sequentielle Ugi-Mehrkomponentenreaktionen, derart hergestellte Verbindungen sowie deren Verwendung als pharmazeutisch verwendbare Produkte, insbesondere als Antibiotika, Antiinfektiva und für alle an die Notwendigkeit zur Zellwandpermeabilität oder Zellwandlokalisation gekoppelten pharmakologischen Anwendungen.

Kondensationsprodukte aus N-substituierten Glycinen (im folgenden Peptoide genannt) zeichnen sich im Vergleich zu ihren verwandten natürlichen Produkten, den Peptiden, durch eine verbesserte metabolische Stabilität aus. Ihre aber häufig vergleichbare biologische bzw. pharmakologische Wirkung macht sie zu interessanten Verbindungen; vgl. beispielsweise J. A. Patch; Pseudo-Peptides in Drug Discovery; (P. E. Nielsen, ed.) WILEY-VCH, Weinheim, 2004.

Peptoide können nach der sog. "sub-monomer"-Methode hergestellt werden. Sie wurde von R. N. Zuckermann ausgearbeitet (vgl. beispielsweise US 2002/115612) und mittlerweile in einer Vielzahl von Arbeiten aufgegriffen Es sind sowohl Applikationen beschrieben, die an fester Phase bzw. eingeschränkt in Lösung durchgeführt werden. Es handelt sich in allen Fällen aber um vielstufige Sequenzen, die den Einsatz geschützter Bausteine notwendig macht. Dabei entstehen Salze als Nebenprodukte. Zudem scheitert häufig die Synthese von Peptoiden, die nicht Polyglycin als Basis haben, oder ist gar unmöglich. So scheitert beispielsweise das Submonomerverfahren zu α,α-disubstituierten Glycinderivaten wie Aminoisobuttersäure (Aib).

Somit liegt der vorliegenden Erfindung die Aufgabe zugrunde, einen einfachen und effizienten Zugang zu derartigen Peptoiden zu ermöglichen, wobei ein solches Syntheseverfahren eine große Vielfalt im Substitutionsmuster gewährleisten soll.

Diese Aufgabe wird durch die in den Ansprüchen gekennzeichneten Ausführungsformen gelöst.

Insbesondere wird ein Verfahren zur Herstellung von Kondensationsprodukten aus N-substituierten Glycinderivaten (Peptoiden) über sequentielle Ugi-Mehrkomponentenreaktionen ("Ugi-4CR") gemäß nachstehendem Schema 1 bereitgestellt: 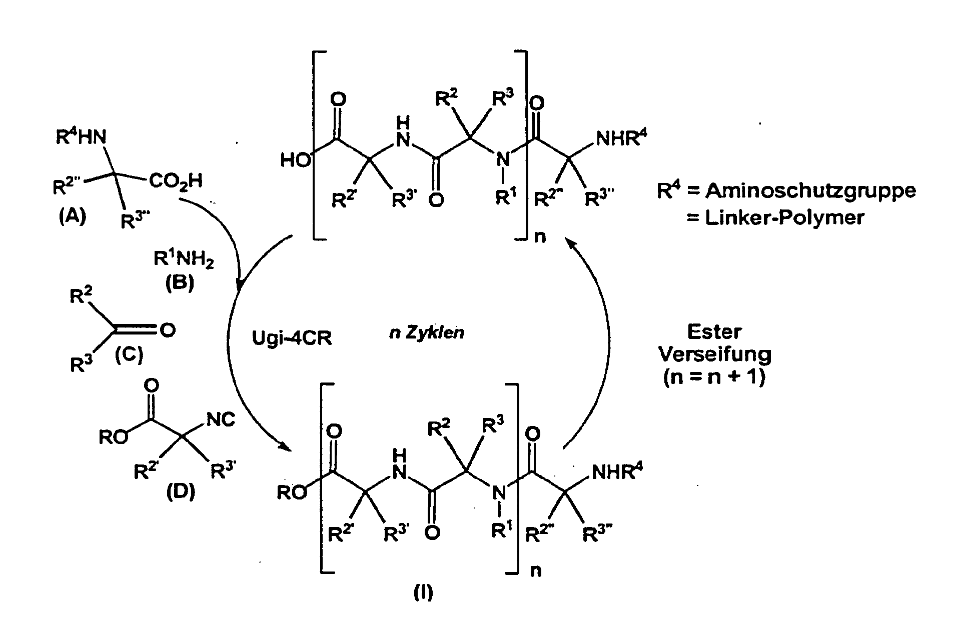 wobei in einem ersten Schritt, vorzugsweise gleichzeitig, ein N-substituiertes Glycinderivat (Komponente A), ein primäres Amin (Komponente B), eine Oxoverbindung (Komponente C) und ein Isonitril (Komponente D), vorzugsweise ein von Glycinestern oder anderen α-Aminosäureestern abgeleitetes Isonitril, umgesetzt werden und nach Esterverseifung des daraus resultierenden Produktes und gegebenenfalls Entfernung von einer oder mehrerer Schutzgruppen die Reaktion n-mal wiederholt wird, wobei das Produkt des jeweiligen vorangegangenen Schritts nach dem ersten Schritt anstelle der Komponente (A) eingesetzt wird, um Verbindungen gemäß der nachstehenden Formel (I) zu erhalten, 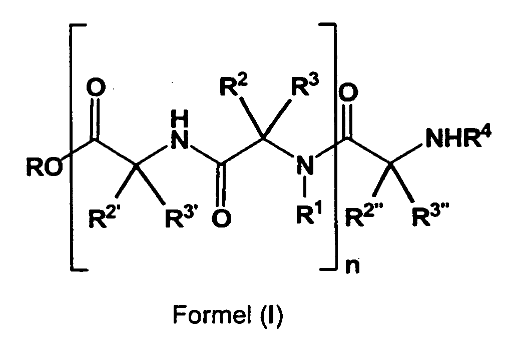 worin
n eine ganze Zahl von 2 bis 100, vorzugsweise 2 bis 50, ist,
die Reste R¹, R^{2,2',2"} und R^{3,3',3"} jeweils unabhängig voneinander H, ein substituierter oder unsubstituierter Alkyl-, Alkoxy-, Aryl-, Cycloalkyl-, Bicycloalkyl-, Tricycloalkyl-, Alkenyl-, Alkinyl-, Alkaryl-, Heteroaryl- mit einem oder mehreren Heteroatomen, ausgewählt aus O, N, S, P, Si oder B, oder Alkheteroarylrest sind, mit der Maßgabe, dass R¹ nicht H ist,
der Rest R⁴ H oder eine abspaltbare Aminoschutzgruppe ist, und der Rest R H oder eine abspaltbare Estergruppe bzw. Carbonsäureschutzgruppe ist.

Das erfindungsgemäße Verfahren gestattet es, mittels repetitiver (iterativer) bzw. konsekutiver Multikomponentenreaktion in einfacher Art und Weise und in vernünftiger Ausbeute derartige Kondensationsprodukte aus N-substituierten Glycinen (Peptoide), d.h. peptidähnliche Poly-N-substituierte Peptide, mit alternierenden Peptid (-CO-NH-) und Peptoid (-CO-NR-) Einheiten herzustellen. Peptoide zeichnen sich durch eine hohe metabolische (Proteasen) Stabilität aus. Sie weisen im Peptidrückgrat aber nur H-Akzeptor-, keine strukturbildenden H-Donor-Eigenschaften auf. Peptide erlauben dagegen bessere Interaktionen und Sekundärstrukturbildung des Polyamidrückgrats mit den Amidgruppen als Wasserstoffbrückendonoren, sie werden aber oft schnell metabolisiert. Die erfindungsgemäß erhältlichen Verbindungen (Kombinationsprodukte) vereinen somit eine erhöhte Proteasestabilität mit H-Donor-Elementen aus Peptiden.

Das erfindungsgemäße Verfahren sieht die Verwendung von Isonitrilen als bifunktionelle Elongationselemente, die sich von Glycinestern oder von anderen α-Aminosäureestern (D-, L-konfiguriert oder racemisch, wobei die Reste R, R^{2'} und R^{3'} wie vorstehend definiert sind) ableiten (Säureelongationsstrategie, Schema 1), vor. Dies bedingt eine Entschützung bzw. Konvertierung der Esterfunktionalität in die für die konsekutive Ugi-Mehrkomponentenreaktion notwendige Carbonsäure. Es können für die Entschützung bzw. Konvertierung klassische Verseifungsmethodiken, aber auch metallkatalysierte und biokatalytische Verfahren verwendet werden, wie sie einem Fachmann wohlbekannt sind.

Im Rahmen des erfindungsgemäßen Syntheseverfahrens ist eine hohe Variabilität der Seitenketten in den Produkten zugänglich. Die gesamte Reaktionsführung ist so gestaltet, dass die Synthese sowohl in Lösung als auch an fester Phase durchgeführt werden kann. Die dadurch zwangsläufig gegebene Erweiterung auf einen hohen Automatisierungsgrad ist eine wesentliche Charakteristik des erfindungsgemäßen Verfahrens.

In einer bevorzugten Ausführungsform wird die Verwendung von festphasengebundenen Carbonsäuren, vorzugsweise Glycinderivaten, die für die Ugi-Mehrkomponentenreaktion die initiale Carboxylatfunktionalität zur Verfügung stellen, vorgesehen.

In jedem Zyklus des erfindungsgemäßen Verfahrens werden unter repetitiver Reaktionsführung mit Säure-Elongationsstrategie dabei zwei Aminosäureeinheiten (Dimereinheiten) hinzugefügt, eine als Peptidbindung, die andere als Peptoidbindung. Möglich wären aber auch zwei Peptidbindungen. Die Reste R¹, R^{2,2'}, R^{3,3'} können dabei unabhängig von denen vorheriger Zyklen gestaltet werden. Bei "Ugireaktiven" Resten sind in den meisten Fällen übliche Schutzgruppen (s. Wuts-Greene, Protective groups in Organic Synthesis, Wiley, 2006; oder Kocienski, Protecting groups, Thieme, 1994) zu verwenden. Dabei ist auf Orthogonalität zur Schutzgruppe R zu achten, was aber einem Fachmann wohlbekannt ist. Eine Ausnahme kann im letzten anzuknüpfenden Dimerelement eintreten, wonach ein komplettes oder partielles Entschützen ermöglicht wird.

Im Rahmen der vorliegenden Erfindung kann auch die Verwendung von Katalysatoren zur Verbesserung der Reaktionen und der Abspaltung der oligomeren bzw. polymeren Produkte von der Matrix, falls die festphasengebundene Synthese gewählt wird, vorgesehen werden. Dieses beinhaltet sowohl die Verwendung von Metall- als auch Biokatalysatoren (Enzymen), wie sie einem Fachmann bekannt sind.

Für das erfindungsgemäße Verfahren können alle herkömmlichen Lösungsmittel, auch Wasser, gegebenenfalls als katalytischer Zusatz auch Mineralsäuren, Lewissäuren und/oder Lewissäurekomplexe, auch chiral und nicht-racemisch, zum Einsatz kommen. Die Synthesereaktionen werden bevorzugt - und abhängig vom Substitutionsmuster - im Bereich von -80 °C - + 200 °C, besonders bevorzugt im Bereich von 0 °C - +50 °C durchgeführt.

Das erfindungsgemäße Verfahren schließt ebenfalls die Verwendung von mikrowellenunterstützter, biokatalytischer und automatisierter Synthese in jedem der Reaktionsschritte ein.

Ein Gegenstand der vorliegenden Erfindung sind somit Peptoidderivate, welche die folgende Struktur gemäß nachstehender Formel (I) aufweisen: 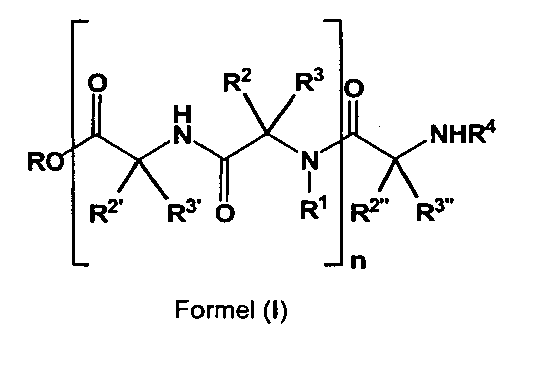 worin
n eine ganze Zahl von 2 bis 100, vorzugsweise 2 bis 50, ist,
die Reste R¹, R^{2,2',2"} und R^{3,3',3"} jeweils unabhängig voneinander H, ein substituierter oder unsubstituierter Alkyl-, Alkoxy-, Aryl-, Cycloalkyl-, Bicycloalkyl-, Tricycloalkyl-, Alkenyl-, Alkinyl-, Alkaryl-, Heteroaryl- mit einem oder mehreren Heteroatomen, ausgewählt aus O, N, S, P, Si oder B, oder Alkheteroarylrest sind, mit der Maßgabe, dass R¹ nicht H ist,
der Rest R⁴ H oder eine abspaltbare Aminoschutzgruppe ist, und der Rest R H oder eine abspaltbare Estergruppe bzw. Carbonsäureschutzgruppe ist.

Vorzugsweise sind die Reste R¹, R^{2,2',2"} und R^{3,3',3"} jeweils unabhängig voneinander aus H, geradkettigen oder verzweigtkettigen (C₁₋₁₂) Alkylresten, geradkettigen oder verzweigtkettigen (C₁₋₁₂) Alkoxyresten, geradkettigen oder verzweigtkettigen (C₁₋₁₂) Trialkylsilylresten, (C₆₋₁₂) Triarylsilylresten, (C₃₋₈) Cycloalkylresten, (C₂₋₁₂) Alkenylresten, (C₂₋₁₂) Alkinylresten, (C₅₋₁₂) Aryl- oder Heteroarylresten, (C₆₋₁₂) Alkaryl- oder Alkheteroarylresten ausgewählt, wobei die Alkyl- oder Arylreste jeweils mit einem oder mehreren von Hydroxy, Alkoxy, Aryloxy, Alkanoyl, Aroyl, Carboxy, Alkoxycarbonyl, Amino, Alkylamino, Aminoalkyl, Hydroxyamino, Amido, Carbamoyl, Ureido, Amidino, Guanidino, Cyano, Azido, Mercapto, Alkylthio, Alkylsulfoxy, Alkylsulfonyl, Alkylsulfenyl, Aminosulfonyl, Sulfhydrylalkyl, Dialkenyldisulfid, Fluor, Chlor, Brom, lod, Alkyl, Perfluoralkyl, Heteroaryl, Polyethylenglycolethyl mit Kettenlänge von 1 bis 40 und Polyethylenglycolmonoether mit Kettenlänge von 1 bis 40 substituiert sein können.

Der hierin verwendete Begriff "Arylrest" unterliegt keiner besonderen Einschränkung und schließt alle chemischen Reste ein, welche ein aromatische Grundgerüst aufweisen, wie beispielsweise eine Phenyl-, Naphthyl- oder Anthranylgruppe. Gemäß der vorliegenden Erfindung schließt der Begriff "Arylrest" sowohl unsubstituierte als auch substituierte aromatische Gruppen ein.

Der Begriff "Alkarylrest", wie in der vorliegenden Erfindung verwendet, schließt all solche Verbindungen ein, welche mit mindestens einer Alkylgruppe substituiert sind, wie beispielsweise Benzyl- oder Ethylphenylgruppen. Dabei kann der "Alkarylrest" sowohl unsubstituiert als auch an einer oder mehreren Alkylgruppen und/oder dem aromatischen Grundgerüst substituiert sein.

Der hierin verwendete Begriff "Heteroarylrest" unterliegt keiner besonderen Beschränkung und schließt alle aromatischen Gruppen ein, deren Grundgerüst ein oder mehrere Heteroatome enthält, wie beispielsweise ein Pyridylrest. Solche Gruppen können unter anderem Derivate von 5-Ringen, wie Pyrrolen, Furanen, Thiophenen oder Imidazolen, oder Derivate von 6-Ringen wie Pyrazinen, Pyridinen oder Pyrimidinen sein.

Der hierin verwendete Begriff "Alkheteroarylrest" unterliegt keiner besonderen Einschränkung und schließt alle Verbindungen ein, welche ein aromatisches Grundgerüst mit mindestens einem Heteroatom und mindestens einer Alkylgruppe enthalten. Beispiel für Alkheteroarylreste sind beispielsweise Picolinylreste.

Durch den konsekutiven Charakter der Reaktionsführung können sich die Reste R¹, R^{2,2',2"} und R^{3,3',3"} in jedem Zyklus voneinander und dadurch auch in den Produkten voneinander unterscheiden und entsprechen den vorstehenden Definitionen für das Substitutionsmuster. Lediglich bei einer vollständig repetitiven Reaktionsführung sind die Reste R¹, R^{2,2',2"} und R^{3,3',3"} in den Produkten identisch.

In einer bevorzugten Ausführungsform sind oder enthalten ein oder mehrere der Reste R¹, R^{2,2',2"} und R^{3,3',3"} Substituenten, die in Größe und Funktionalität proteinogenen Aminosäureseitenketten, wie beispielsweise CH₂-OH und CH₂SH für Serin und Cystein, vergleichbar sind bzw. insbesondere durch N-haltige und lipophile Alkylketten gekennzeichnet sind, oder große lipophile Reste wie tert.-Butyl, Phenyl, Naphthyl bzw. Anthranyl sind. In einer weiteren Ausführungsform der vorliegenden Erfindung sind ein oder mehrere der Reste R¹, R^{2,2',2"} und R^{3,3',3"}, insbesondere ein oder mehrere von R^{2,2',2"} und R^{3,3',3"}, Methylgruppen (→ Dimethylsubstitution analog Aminoisobuttersäure als Baustein).

Besonders bevorzugt sind in der Oxokomponente R² = R³ =H (d.h. Komponente (C) ist Paraformaldehyd). In einer anderen Ausführungsform ist R³ H und R² eine aminosäureanaloge Seitenkette, z.B. proteinogene Aminosäureseitenketten wie beispielsweise CH₂-OH und CH₂SH für Serin und Cystein, bzw. N-haltige und lipophile Alkylketten oder große lipophile Reste wie tert.-Butyl, Phenyl, Naphthyl bzw. Anthranyl, alle in racemischer bzw. chiraler, nicht-racemischer Form. Im Rahmen des erfindungsgemäßen Verfahrens kann die Oxokomponente auch als Acetal geschützt eingesetzt werden, wenn eine saure Reaktionsführung in Gegenwart von Wasser erfolgt.

Alle vorstehend aufgeführten Reste, die funktionelle Gruppen mit Ugi-Reaktivität besitzen, insbesondere primäre Amine, Carbonsäuren, Aldehyde, je nach Reaktivität ggf. auch Ketone, und Guanidine, müssen gemäß üblicher Verfahren (Wuts-Greene, Protective Groups in Organic Synthesis, Wiley, 2006; oder Kocienski, Protecting groups) geschützt werden. Die Schutzgruppen sind dabei so zu wählen, dass sie im Verlaufe der Elongation (insbesondere der Esterentschützung) nicht ungewollt freigesetzt werden (Auswahl orthogonaler Schutzgruppen), z.B. in Analogie zu den bekannten Verfahren der Festphasenpeptidsynthese.

Der Rest R steht für H oder eine übliche, abspaltbare Estergruppe (Schutzgruppe der Carbonsäurefunktionalität der Komponente (D)), insbesondere Alkyl, Aryl, Cycloalkyl, Cylcoaryl, Heterozyklen und Alkylheterocyclen, enthaltend eines oder mehrere von O, N, S, P Si oder B, vorzugsweise für geradkettige oder verzweigtkettige (C₁₋₁₂) Alkylreste, (C₃₋₈) Cycloalkylreste, (C₅₋₁₂) Aryl- oder Heteroarylreste, (C₆₋₁₂) Alkaryl- oder Alkheteroarylreste. Bevorzugt ist ein Rest der gemäß Wuts-Greene, Protective Groups in Organic Synthesis, Wiley, 2006; oder Kocienski, Protecting groups, Thieme, 1994, als spaltbare Esterschutzgruppe geeignet ist, auch photospaltbare Schutzgruppen. Beispielhafte abspaltbare Estergruppen, die als Rest R in dem erfindungsgemäßen Verfahren verwendet werden können, sind Methyl und Ethyl.

Der Rest R⁴ ist eine gebräuchliche Aminoschutzfunktionalität (Wuts-Greene, Protective groups in OrganicSynthesis, Wiley, 2006; Kocienski, Protecting groups, Thieme, 1994) oder aber eine über einen Linker verknüpfte polymere Phase, die für Festphasensynthesen eingesetzt werden (S. Miertus, CRC, 2006; A. W. Czarnik, CRC, 2002), bevorzugt solche, die auch in der Peptidchemie Anwendung finden. Beispielhafte Aminoschutzgruppen, die als Rest R⁴ in dem erfindungsgemäßen Verfahren verwendet werden können, sind Carbamat-enthaltend wie z.B. CBz (Benzoyloxycarbonyl), aktivierte Amide (z.B. Indolyl-Amide) oder Azide.

Bevorzugte Peptoidderivate, welche die Struktur gemäß vorstehender Formel (I) aufweisen, sind erfindungsgemäß wie folgt:

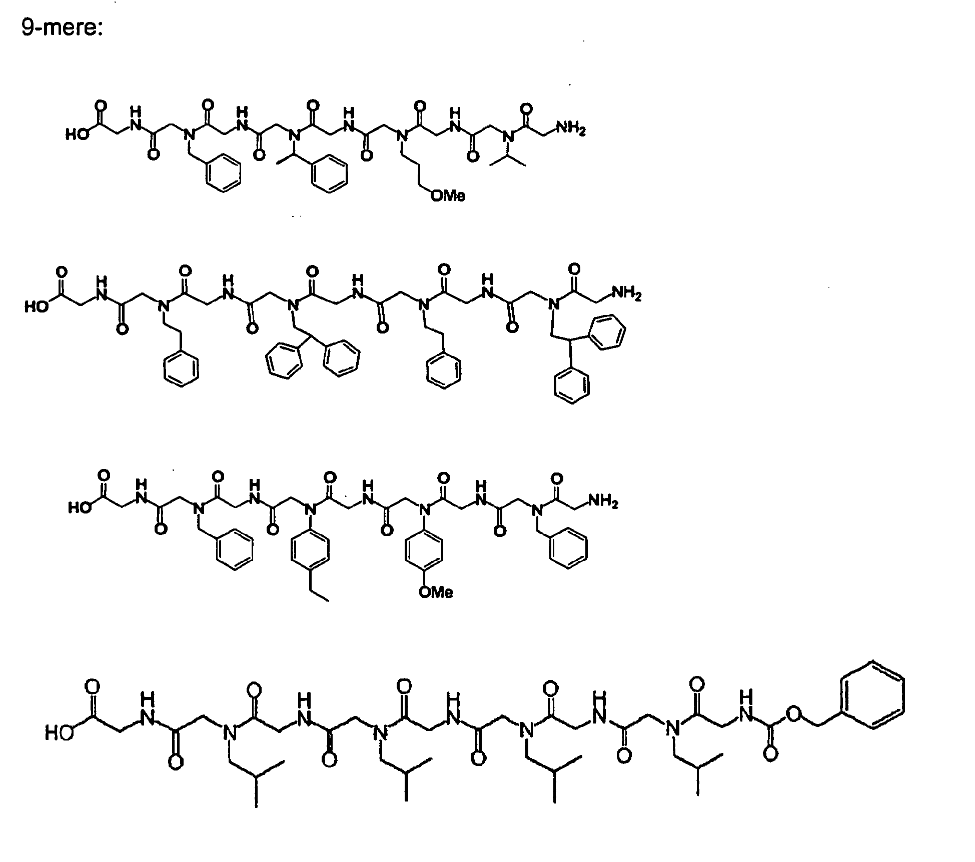

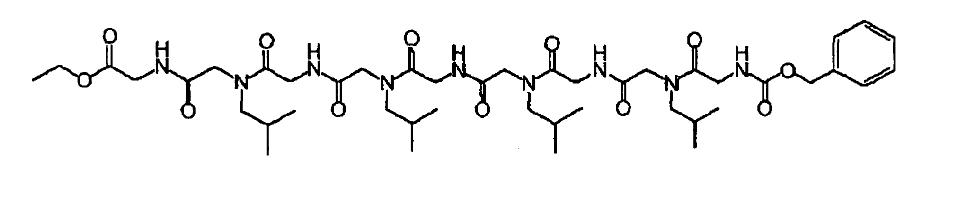

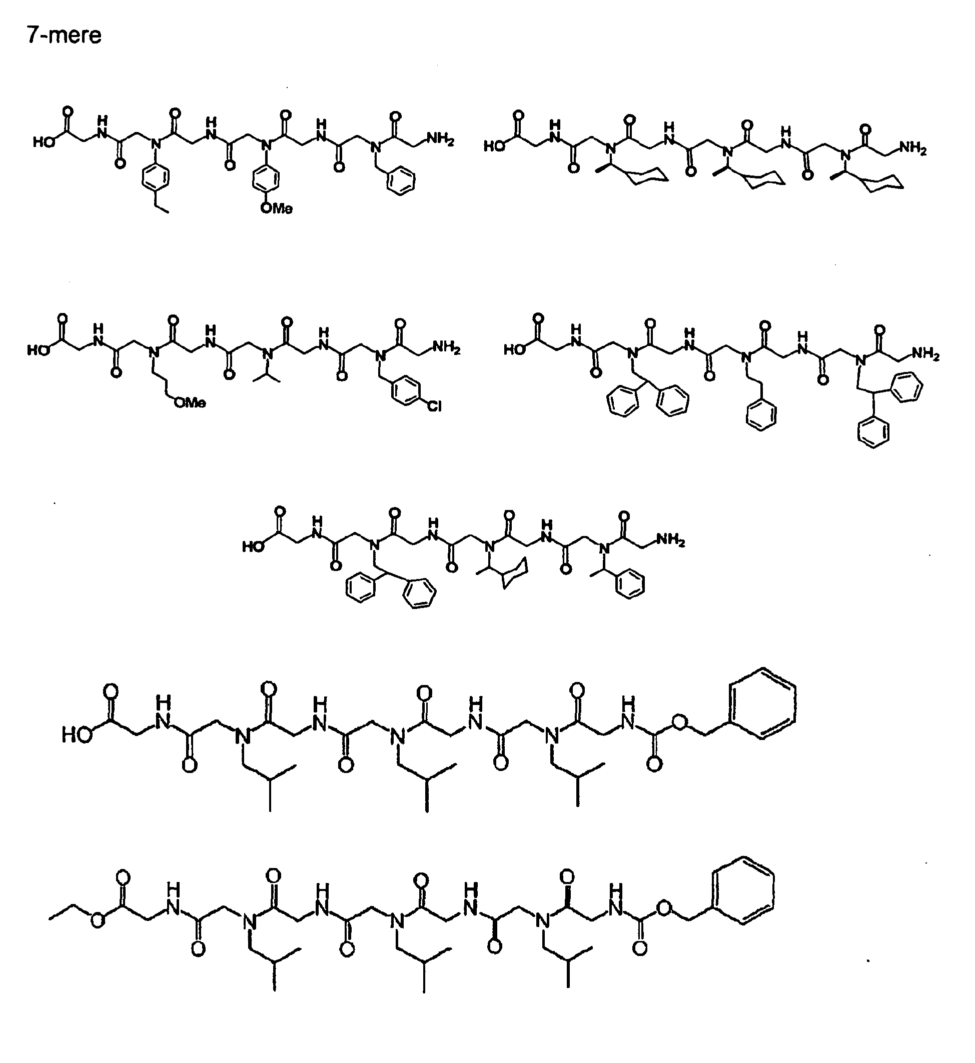

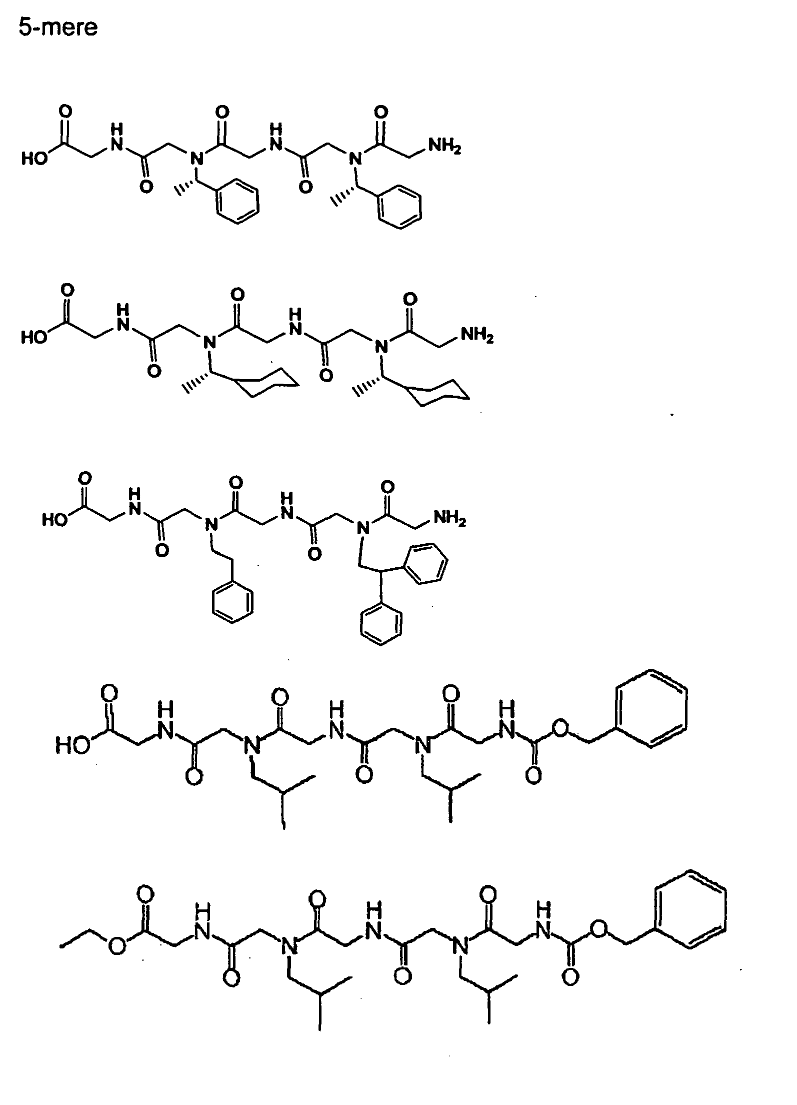

Weitere bevorzugte Peptoidderivate weisen folgende Struktur auf: 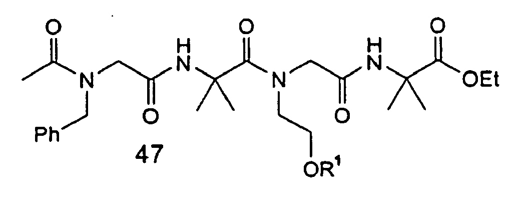 wobei R¹ wie vorstehend definiert ist, insbesondere H oder TBS ist, sowie

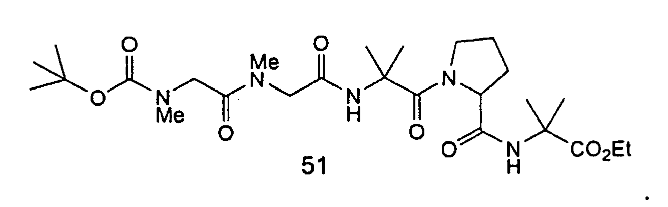

Im Rahmen der vorliegenden Erfindung wurde festgestellt, dass die durch das erfindungsgemäße Verfahren erhältlichen Verbindungen in folgenden Indikationsfeldern eingesetzt werden können:
- als Biozide, besonders als Antibiotika und Antiinfektiva, insbesondere als Antibiotika mit ungewöhnlichem oder individuell angepaßtem Wirkprofil (z.B. selektiv für gramnegative Bakterien).
- als Konjugate mit bekannten Wirkstoffen oder Indikator- oder Reportereinheiten (Farbstoffe, Spinlabel etc.) zum Zweck des aktiven und passiven Transports durch Zellmembranen oder zum spezifischen Binden in Zellmembranen.

Die erfindungsgemäß erhältlichen Verbindungen können auch als pharmazeutische bzw. pharmakologische Präparationen bzw. Zusammensetzungen vorgesehen werden. Beispiele für pharmakologisch akzeptable Salze der erfindungsgemäß erhältlichen Verbindungen der Formel (I) sind Salze (oder Mischsalze) von physiologisch verträglichen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Verbindungen der Formel (I) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) auftreten. Wenn die Verbindungen der Formel (I) asymmetrische C-Atome enthalten, können sie entweder als achirale Verbindungen, Diastereomeren-Gemische, Gemische von Enantiomeren oder als optisch reine Verbindungen vorliegen. Im Rahmen der vorliegenden Erfindung sind auch alle cis/trans-Isomeren der Verbindungen der allgemeinen Formel (I) sowie Gemische davon umfasst.

Solche pharmazeutischen Zusammensetzungen, die mindestens eine Verbindung der allgemeinen Formel (I) als Wirkstoff aufweisen, enthalten üblicherweise weiter ein oder mehrere pharmakologisch verträgliche Trägerstoffe und/oder Adjuvantien.

Die vorliegende Erfindung betrifft auch entsprechende Pro-Drugs der Verbindungen der allgemeinen Formel (I). Die Pro-Drugs der Verbindungen (siehe beispielsweise R. B. Silverman, Medizinische Chemie, VCH Weinheim, 1995, Kapitel 8, S. 361ff) bestehen aus einer Verbindung der Formel (I) und mindestens einer pharmakologisch akzeptablen Schutzgruppe, die unter physiologischen Bedingungen abgespalten wird, z.B. einer Alkoxy-, Aralkyloxy-, Acyl- oder AcyloxyGruppe, wie z.B. einer Ethoxy-, Benzyloxy-, Acetyl- oder Acetyloxy-Gruppe. Konjugate bestehen analog aus Verbindungen der Formel (I), einem physiologisch spaltbaren Linker (bifunktionell analog der vorgenannten Prodrug-Schutzgruppen) oder stabilen Linkern, z.B. Alkyliden und Polyetherlinker, insbesondere PEG-Linker.

Ein weiterer Gegenstand der vorliegenden Erfindung betrifft die therapeutische oder diagnostische Verwendung der Verbindungen der Formel (I), ihrer pharmakologisch verträglichen Salze bzw. Solvate und Hydrate sowie Formulierungen und pharmazeutischen Zusammensetzungen, insbesondere die Verwendung dieser Wirkstoffe zur Herstellung von Arzneimitteln zur Behandlung von Infektionen und zur Diagnostik. Im allgemeinen werden Verbindungen der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit einem oder mehreren pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch, etc.. Zur Herstellung von weichen Kapseln können Arzneimittelträgerstoffe wie z.B. pflanzliche Öle, Petroleum, tierische oder synthetische Öle, Wachs, Fett, Polyole eingesetzt werden. Zur Herstellung von flüssigen Lösungen und Sirups können Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwendet werden. Für Suppositorien können Arzneimittelträgerstoffe wie z.B. pflanzliche Öle, Petroleum, tierische oder synthetische Öle, Wachs, Fett und Polyole verwendet werden. Für Aerosol-Formulierungen können komprimierte Gase, die für diesen Zweck geeignet sind, wie z.B. Sauerstoff, Stickstoff, Edelgase und Kohlendioxid, eingesetzt werden. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Anti-oxidantien enthalten.

Kombinationen mit anderen therapeutischen und/oder diagnostischen Mitteln können weitere Wirkstoffe beinhalten, die gewöhnlich zur Behandlung von Infektionen oder zur Diagnostik oder Minderung von dabei auftretenden Effekten (z. B. Antiallergika) eingesetzt werden.

Zur Behandlung von Krebserkrankungen kann die Dosis der erfindungsgemäßen, biologisch oder diagnostisch aktiven Verbindung innerhalb breiter Grenzen variieren und kann auf den individuellen Bedarf eingestellt werden. Im allgemeinen ist eine Dosis von 1 µg bis 1000 mg/kg Körpergewicht pro Tag geeignet, wobei eine bevorzugte Dosis 10 µg bis 25 mg/kg pro Tag ist. In geeigneten Fällen kann die Dosis auch unter oder über den oben angegebenen Werten liegen.

Die folgenden Beispiele dienen dazu, die vorliegende Erfindung weiter zu veranschaulichen, ohne diese in irgendeiner Weise einzuschränken.

Allgemeine Arbeitsvorschrift

### Schritt a: Ugi-4-Komponenten Kondensationsreaktion

Paraformaldhyd (2 mmol), Amin (2 mmol) und Natriumsulfat in 25 ml Alkohol (genaure Angaben im spezifischen Experiment) werden bei Raumtemperatur gemischt und für 2,5 h gerührt. Nach dem Zugeben der Säure (1 mmol) wird die Reaktionsmischung für weitere 30 min bei Raumtemperatur belassen. Nach Zugabe des Isonitrils (1 mmol) wird bei Raumtemperatur ein bis drei Tage gerührt. Nach Filtration der Suspension wird im Vakuum eingeengt und der Rückstand durch Flash Chromatographie an Kieselgel (Laufmittel CH₂Cl₂ mit 1 - 5% Methanol).

### [(Benzyloxy)carbonyl]glycyl-N-isobutylglycylglycinatethylester (Peptoid 37)

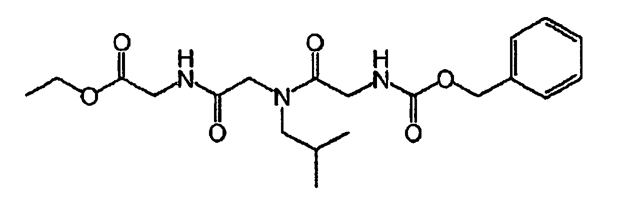

Allgemeine Arbeitsvorschrift a, eingesetzte Mengen: Isobutylamin (2.0 g, 23.6 mmol), Paraformaldehyd (0,82 g, 23,6 mmol), N-Benzoyloxycarbonyl-glycin (2,84 g, 13,6 mmol) und Isocyanoessigsäureethylester (1,53 g, 13,6 mmol) in Methanol (100 ml). Nach 24 h wurde die Reaktion aufgearbeitet und durch Flashchromatographie gereinigt (3,5 x 30 cm, CH₂Cl₂/MeOH 97 : 3).
Ausbeute 3,32 g (8,2 mmol, 60 %), farbloses Öl.
Rf: 0,30 (CH₂Cl₂/MeOH 9 : 1).
MS (ESI-MS): m/z (%) = 430 (100) [M+Na]⁺, 408,6 (15) [M+H]⁺.
HRMS-ESI ber. C₂₀H₂₉N₃O₆Na [M+Na]⁺: 430,4215, gef. 430,4213.

### N-[(Benzyloxy)carbonyl]glycyl-N-isobutylglycylglycin (Peptoid 38)

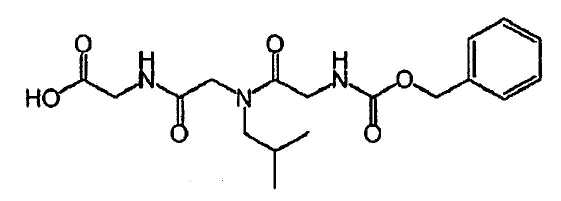

Peptoid **37** (3,74 g, 9,2 mmol) wird mit LiOH x H₂O (0.96 g, 23 mmol) in THF/H₂O (30 ml, 2 : 1 v/v) bei Raumtemperatur versetzt. Nach der Neutralisation und Filtration wird das Produkt durch Kristallistisation (EtOAc/Petrolether 3 : 2) gereinigt und getrocknet.
Ausbeute: 3,14 g (8,3 mmol, 90 %), weißer Feststoff.
R_{f} 0,25 (CH₂Cl₂/MeOH 8 : 2).
Smp 102 - 104 °C.
MS (ESI-MS): m/z (%) = 402 (100) [M+Na]⁺, 380,2 (2) [M+H]⁺.
HRMS-ESI ber. C₁₈H₂₅N₃O₆Na [M+Na]⁺: 402,1635, gef. 402,1627.

### N-[(Benzyloxy)carbonyl]glycyl-N-isobutylglycylglycyl-N-isobutylglycylglycinethylester (Peptoid 39)

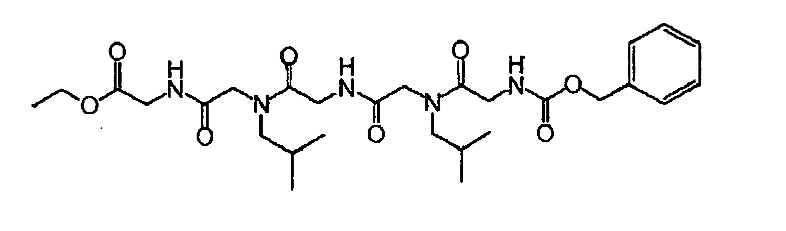

Allgemeine Arbeitsvorschrift a, eingesetzte Mengen: Isobutylamin (0,79 g, 10,8 mmol), Paraformaldehyd (0,82 g, 23,6 mmol), Peptoid 38 (2,05 g, 5,4 mmol), Isocyanoessigsäureethylester (0,61 g, 5,4 mmol) in Methanol (250 ml). Aufarbeitung nach 48 h, Aufreinigung durch Flashchromatographie (2,5 x 30 cm, CH₂Cl₂/MeOH 95 : 5).

| | |
|---|---|
| Ausbeute | 2,1 g (3,6 mmol, 67 %), farbloser Feststoff |
| R_{f} | 0,25 (CH₂Cl₂/MeOH 95 : 5). |
| Smp | 98 - 102 °C. |

MS (ESI-MS): m/z (%) = 600.1 (100) [M+Na]⁺, 578.8 (35) [M+H]⁺.
HRMS-ESI ber. C₂₈H₄₃N₅O₅Na [M+Na]⁺: 600,3003, gef. 600,2999.

### N-[(Benzyloxy)carbonyl]glycyl-N-isobutylglycylglycyl-N-isobutylglycylglycin (Peptoid 40)

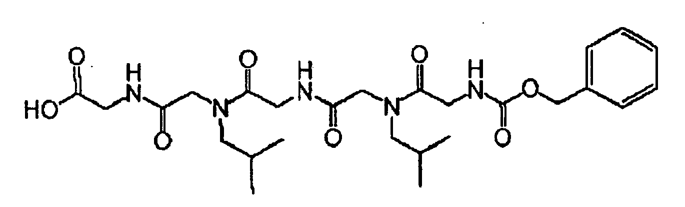

Vorschrift: analog Peptoid **38,** eingesetzte Mengen: Peptoid **39** (0,2 g, 0,34 mmol), LiOH x H₂O (0,036 g, 0,86 mmol) in THF/H₂O (3 ml, 2 : 1 v/v).

| | |
|---|---|
| Ausbeute | 0,19 g (0.34 mmol, 96 %), farbloser Feststoff |
| R_{f} | 0,28 (CH₂Cl₂/MeOH 8 : 2). |
| Smp | 87 - 90 °C. |

MS (ESI-MS): m/z (%) = 572,3 (100) [M+Na]⁺, 550,7 (20) [M+H]⁺.
HRMS-ESI ber. C₂₆H₃₉N₅O₈Na [M+Na]⁺: 572,2690, gef. 572,2688.

### N-[(Benzyloxy)carbonyl]glycyl-N-isobutylglycylglycyl-N-isobutylglycylglycyl-N-isobutylglycylglycinethylester (Peptoid 41)

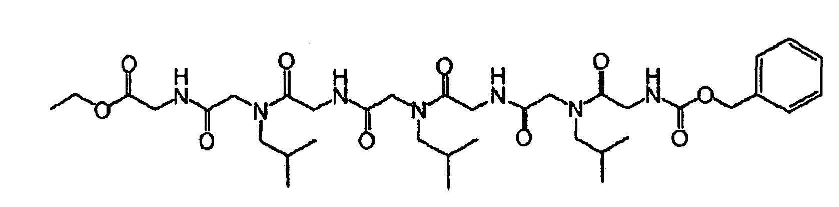

Allgemeine Arbeitsvorschrift a, eingesetzte Mengen: Isobutylamin (0,53 g, 7,28 mmol), Paraformaldehyd (0,21 g, 7,28 mmol), Peptoid **40** (2,0 g, 3,64 mmol), Isocyanoessigsäureethylester (0,41 g, 3,64 mmol) in Trifluorethanol (50 ml). Aufarbeitung nach 48 h, Aufreinigung durch Flashchromatographie (3,5 x 30 cm, CH₂Cl₂/MeOH 95 : 5).
Ausbeute 1,73 g (2,3 mmol, 64 %), gelbliches Öl.
MS (ESI-MS): m/z (%) = 770,4 (100) [M+Na]⁺, 748,4 (10) [M+H]⁺.
HRMS-ESI ber. C₃₆H₅₇N₇O₁₀Na [M+Na]⁺: 770,4059, gef. 770,4034.

### N-[(Benzyloxy)carbonyl]glycyl-N-isobutylglycylglycyl-N-isobutylglycylglycyl-N-isobutylglycylglycin (Peptoid 42)

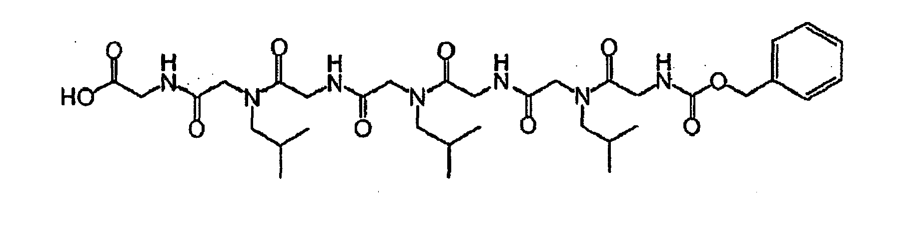

Vorschrift: analog Peptoid **38,** eingesetzte Mengen: Peptoid **41** (0,38 g, 0,51 mmol), LiOH x H₂O (0,054 g, 1.27 mmol) in THF/H₂O (15 ml, 2:1 v/v). Aufreinigung durch Flashchromatographie an Kieselgel (2,5 x 30 cm, CH₂Cl₂/MeOH 7 : 3).
Ausbeute 0,30 g (0,43 mmol, 85 %), gelbliches Öl
R_{f} 0,35 (CH₂Cl₂/MeOH 6 : 4).
MS (ESI-MS): m/z (%) = 742,7 (80) [M+Na]⁺.
HRMS-ESI ber. C₃₄H₅₃N₇O₁₀Na [M+Na]⁺: 742,3746, gef. 742,3735.

### N-[(Benzyloxy)carbonyl]glycyl-N-isobutylglycylglycyl-N-isobutylglycylglycyl-N-isobutylglycylgylcyl-N-isobutylglycylglycinethylester (Peptoid 43)

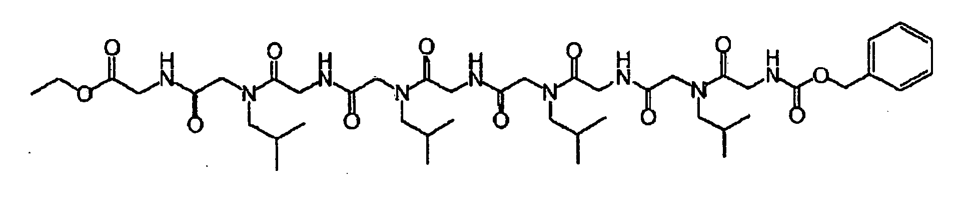

Allgemeine Arbeitsvorschrift a, eingesetzte Mengen: Isobutylamin (0,04 g, 0,55 mmol), Paraformaldehyd (0,016 g, 0,55 mmol), Peptoid **42** (0,2 g, 0,28 mmol), Isocyanoessigsäureethylester (0,03 g, 0,28 mmol) in Ethanol (5 ml). Aufarbeitung nach 72 h, Aufreinigung durch HPLC RP₁₈ (Lösungsmittel A: H₂O; Lösungsmittel B: CH₃CN, Gradient 20 - 60 %, Flussrate 20 ml/min).

| | |
|---|---|
| Ausbeute | 0,13 g (0,15 mmol, 52 %), gelbliches Öl. |
| R_{f} | 0,35 (CH₂Cl₂/MeOH 9 : 1). |

MS (ESI-MS): m/z (%) = 941,0 (100) [M+Na]⁺, 918,8 (35) [M+H]⁺.
HRMS-ESI ber. C₄₄H₇₁N₉O₁₂Na [M+Na]⁺: 940,5114, gef. 940,5097.

### N-[(Benzyloxy)carbonyl]glycyl-N-isobutylglycylglycyl-N-isobutylglycylglycyl-N-isobutylglycylglycyl-N-isobutylglycylglycin (Peptoid 44).

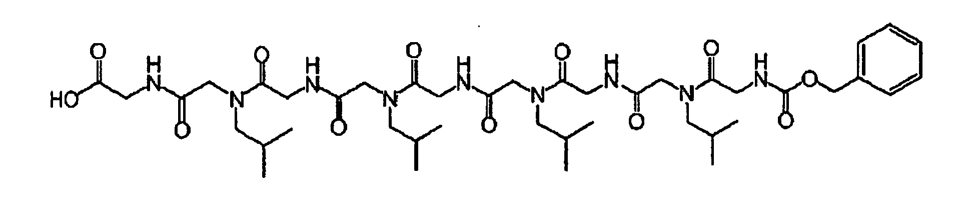

Vorschrift: analog Peptoid **38,** eingesetzte Mengen: Peptoid **43** (0,03 g, 0,032 mmol), LiOH x H₂O (3,4 mg, 0,08 mmol) in THF/H₂O (4 ml, 2 : 1 v/v). Aufreinigung durch Flashchromatographie an Kieselgel (2,0 x 20 cm, CH₂Cl₂/MeOH 6 : 4).

| | |
|---|---|
| Ausbeute | 27 mg (0,032 mmol, 94 %), gelbliches Öl. |
| R_{f} | 0,30 (CH₂Cl₂/MeOH 6 : 4). |

MS (ESI-MS): m/z (%) = 934 (100) [M+Na]⁺, 912,8 (100) [M+H]⁺.
HRMS-ESI ber. C₄₂H₆₇N₉O₁₂Na [M+Na]⁺: 912,4801, gef. 912,4790.

Biologisches Screening: Die vorstehend angeführten, erfindungsgemäß hergestellten Derivate wiesen bis > 1000 µM keinen nennenswerten Einfluß auf das Wachstum des grampositiven Keims *Bacillus subtilis.* Gramnegatives *Vibrio fischeri* (Bioluminiszenzassay) war gegen die vorstehend angeführten, erfindungsgemäß hergestellten Derivate dagegen empfindlich, insbesondere gegen folgendes Peptid-Peptoid (mit IC-50 ca. 10 µM).

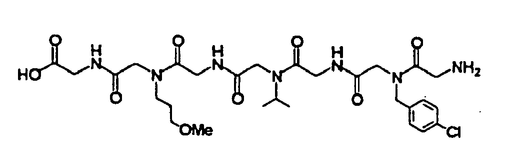

### Synthese von AIB (Aminoisobuttersäure)-enthaltenen Peptoiden

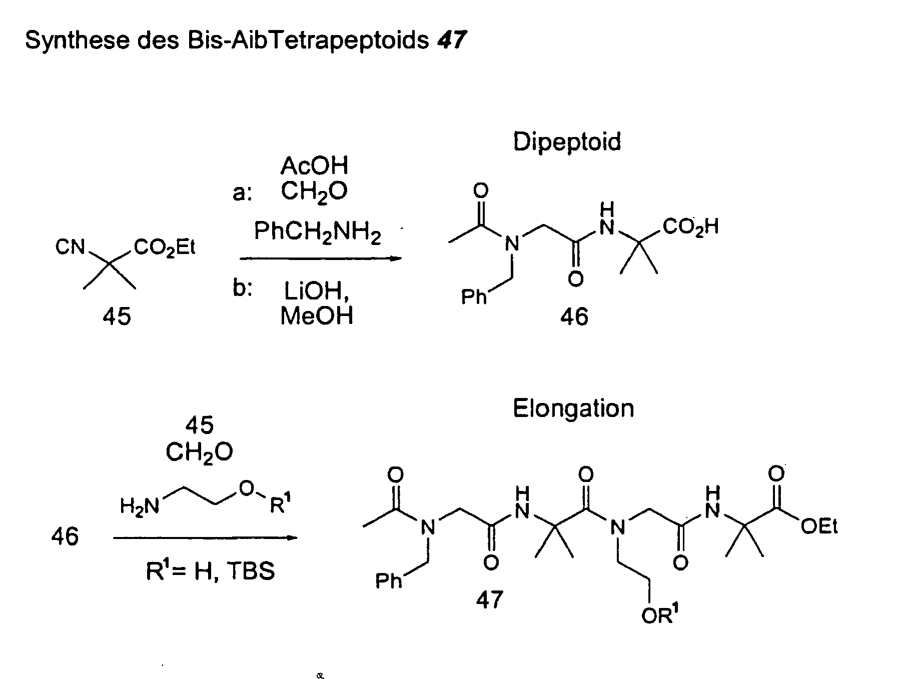

Allgemeine Arbeitsvorschrift a, eingesetzte Mengen: Benzylamin (1,07g, 10 mmol), Paraformaldehyd (0,33g, 11 mmol), AIB-Isonitril **45** (1,4g, 10 mmol) und Essigsäure (0,6g, 10 mmol) 2 ml CH₂Cl₂ und 1 ml MeOH. Nach 20 h (TLC-Kontrolle R_{f}^{prod}= 0,2 (CHCl₃: MeOH = 20:1)) wurde das Reaktionsgemisch aufgearbeitet. Der Rückstand wurde in MeOH (20 ml) und 5M LiOH (10 ml) aufgenommen und 3 h gerührt. Nach Einengen der Lösung unter vermindertem Druck wurde 1 N HCI zugegeben und bei 4 °C gekühlt. Der Niederschlag wurde filtriert und getrocknet. Die Ausbeute an **46** beträgt 1,8 g (62%).
¹H NMR (D6-DMSO) (s-cis/s-trans Isomere, 2:1) δ (ppm) = 1,32, 1,33 (s,s, 6H, (CH₃)₂), 2,01, 2,06 (s, s, 3H, CH₃), 3,85 (s, 2H, CH₂), 4,42, 4,53 (s,s, 2H, CH₂Ph), 7,29 (m, 5H, Ph), 8,06, 8,27 (s,s, 1H, NH).

Für die Elongation allgemeine Arbeitsvorschrift a, eingesetzte Mengen: Säure **46,** Amin (1 eq.), Paraformaldehyd (1,5 eq.) und Isonitril **45** (1 eq.) in MeOH. Die Reaktionsmischung wird für 24 h gerührt. Nach Kontrolle der Reaktionsmischung mittels LCMS wird nach dem Standardverfahren aufgearbeitet. Aufgrund der cis/trans Isomeren sind die NMR-Signale mehrfach aufgespalten.
LCMS **47:** M+H⁺ m/z = 507,3 (R = H)
M+H⁺ m/z = 621,4 (R = TBS).

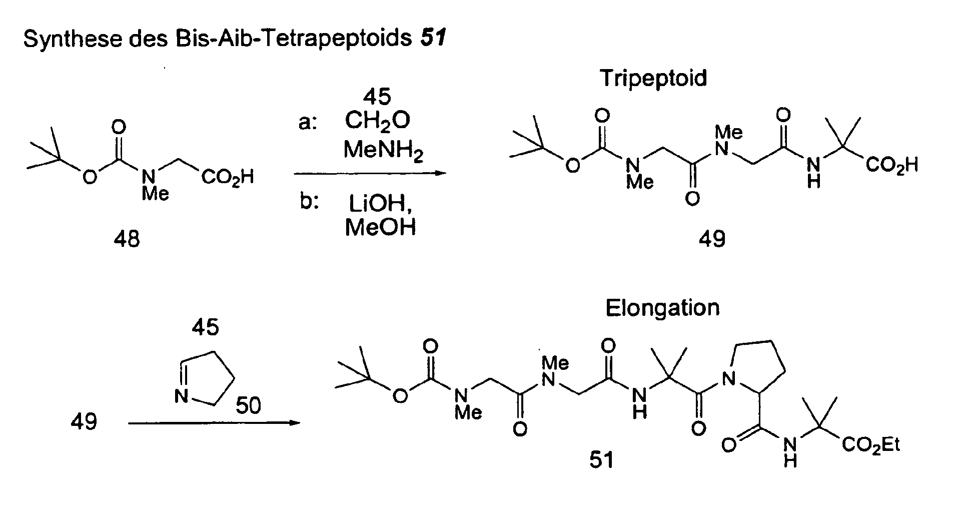

Allgemeine Arbeitsvorschrift a, eingesetzte Mengen: Methylamin (33 %, 740 mg), Paraformaldehyd (237 mg, 7,90 mmol) in MeOH/CH₂Cl₂ (6 ml, 1:2), Bocgeschütztes Sarcosin **48** (1,5 g, 7,9 mmol) in MeOH (2 ml) und Isonitril **45** (1,1 g, 7,8 mmol). Nach 12 h Rühren bei Raumtemperatur wird unter vermindertem Druck eingeengt und das verbleibende Gemisch in MeOH (40 ml) aufgenommen.
Nach Zugabe von 5 M LiOH (7,0 ml) wird noch 3 h bei Raumtemperatur gerührt (LCMS-Kontrolle), um dann die Aufarbeitung durch Einengen des Lösungsmittels vorzunehmen. Lösen in 1 N HCl und Extraktion mit Essigester führt zu **49** in 70 % Ausbeute (1,90 g). Die NMR-Signale sind aufgrund der *cis*/*trans* Isomerie sehr stark aufgespalten.
LCMS **49:** M+H⁺ = 346
M-H⁻ = 344 (M = 345). Reinheit > 95%.

Im Handel erhältlicher 4-Aminobutyraldehyd (12 g) wird mit 2 M HCl (75 ml) für 2 h bei 0 °C gerührt, bevor langsam 2,67 M K₂CO₃-Lösung (225 ml) hinzugegeben wird. Nach Rühren für 1 h bei Raumtemperatur wird mit CH₂Cl₂ (3 x 200 ml) extrahiert, Einengen des Lösungsmittels ergab ein farbloses Öl (9,1 g), das nach fraktionierter Destillation das Imin **50** ergab.

Zu einer Lösung des Imins **50** (1 eq.) in Trifluorethanol (ca. 10 ml/g) werden anschließend die Carbonsäure **49** und das Isonitril **45** (1 eq.) gegeben. Rühren für 20 h bei 90 °C (LCMS-Kontrolle), Aufarbeitung und Abtrennung von nicht reagiertem Startmaterial ergab das Tetrapeptoid **51.**
ESI-MS **51:** m/z = 556,3 (M+H)

## Patentansprüche

1. Verfahren zur Herstellung von Kondensationsprodukten aus N-substituierten Glycinderivaten (Peptoiden) über sequentielle Ugi-Mehrkomponentenreaktionen gemäß nachstehendem Schema 1: 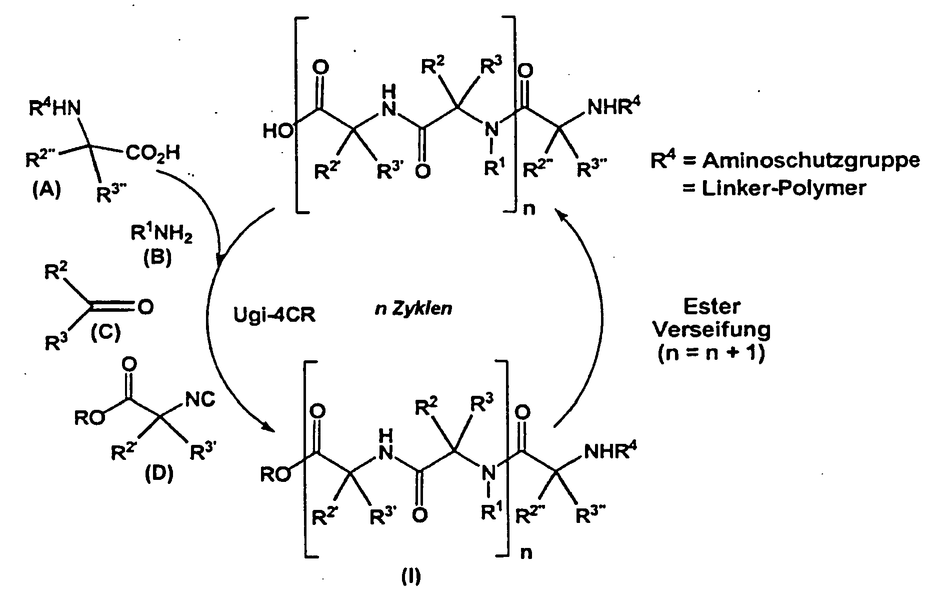 wobei in einem ersten Schritt, vorzugsweise gleichzeitig, ein N-substituiertes Glycinderivat (Komponente A), ein primäres Amin (Komponente B), eine Oxoverbindung (Komponente C) und ein Isonitril (Komponente D) umgesetzt werden und nach Esterverseifung des daraus resultierenden Produktes und gegebenenfalls Entfernung von einer oder mehrerer Schutzgruppen die Reaktion n-mal wiederholt wird, wobei das Produkt des jeweiligen vorangegangenen Schritts nach dem ersten Schritt anstelle der Komponente (A) eingesetzt wird, um Verbindungen gemäß der nachstehenden Formel (I) zu erhalten, 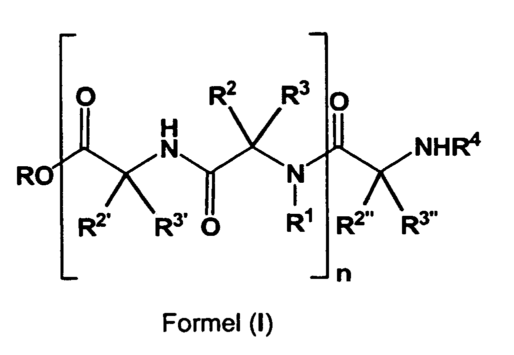 worin
n eine ganze Zahl von 2 bis 100 ist,
die Reste R¹, R^{2,2',2"} und R^{3,3',3"} jeweils unabhängig voneinander H, ein substituierter oder unsubstituierter Alkyl-, Alkoxy-, Aryl-, Cycloalkyl-, Bicycloalkyl-, Tricycloalkyl-, Alkenyl-, Alkinyl-, Alkaryl-, Heteroaryl- mit einem oder mehreren Heteroatomen, ausgewählt aus O, N, S, P, Si oder B, oder Alkheteroarylrest sind, mit der Maßgabe, dass R¹ nicht H ist,
der Rest R⁴ H oder eine abspaltbare Aminoschutzgruppe ist, und
der Rest R H oder eine abspaltbare Estergruppe bzw. Carbonsäureschutzgruppe ist.

2. Verfahren gemäß Anspruch 1, worin die Komponente (D) ein von Glycinester oder Aminoisobuttersäureester abgeleitetes Isonitril ist.

3. Verfahren gemäß Anspruch 1 oder 2, worin die Komponente (B) Paraformaldehyd ist.

4. Verfahren gemäß einem der Ansprüche 1 bis 3, wobei als Komponente (A) eine festphasengebundene Carbonsäure, die für die Ugi-Mehrkomponentenreaktion die initiale Carboxylatfunktionalität zur Verfügung stellen, eingesetzt wird.

5. Verfahren gemäß einem der Ansprüche 1 bis 4, wobei die Reste R¹, R^{2,2',2"} und R^{3,3',3"} jeweils unabhängig voneinander aus H, geradkettigen oder verzweigtkettigen (C₁₋₁₂) Alkylresten, geradkettigen oder verzweigtkettigen (C₁₋₁₂) Alkoxyresten, geradkettigen oder verzweigtkettigen (C₁₋₁₂) Trialkylsilylresten, (C₆₋₁₂) Triarylsilylresten, (C₃₋₈) Cycloalkylresten, (C₂₋₁₂) Alkenylresten, (C₂₋₁₂) Alkinylresten, (C₅₋₁₂) Aryl- oder Heteroarylresten, (C₆₋₁₂) Alkaryl- oder Alkheteroarylresten ausgewählt sind, wobei die Alkyl- oder Aryl-Reste jeweils mit einem oder mehreren von Hydroxy, Alkoxy, Aryloxy, Alkanoyl, Aroyl, Carboxy, Alkoxycarbonyl, Amino, Alkylamino, Aminoalkyl, Hydroxyamino, Amido, Carbamoyl, Ureido, Amidino, Guanidino, Cyano, Azido, Mercapto, Alkylthio, Alkylsulfoxy, Alkylsulfonyl, Alkylsulfenyl, Aminosulfonyl, Sulfhydrylalkyl, Dialkenyldisulfid, Fluor, Chlor, Brom, lod, Alkyl, Perfluoralkyl, Heteroaryl, Polyethylenglycolethyl mit Kettenlänge von 1 bis 40 und Polyethylenglycolmonoether mit Kettenlänge von 1 bis 40 substituiert sein können.

6. Verfahren gemäß einem der Ansprüche 1 bis 5, wobei die Reste R Methyl oder Ethyl ist.

7. Verfahren gemäß einem der Ansprüche 1 bis 6, wobei der Rest R⁴ Benzoyloxycarbonyl ist.

8. Verfahren gemäß einem der Ansprüche 1 bis 7, wobei die Umsetzung im Bereich von -80 °C - + 200 °C , besonders bevorzugt im Bereich von 0 °C - +50 °C durchgeführt wird.

9. Peptoidderivate, welche die Struktur gemäß nachstehender Formel (I) aufweisen: 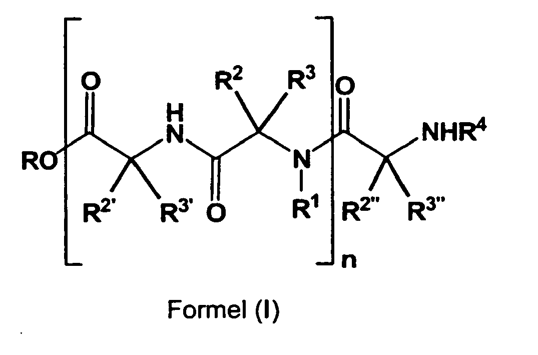 worin
n, R¹, R^{2,2',2"}, R^{3,3',3"}, R⁴ und R wie vorstehend definiert sind.

10. Peptoidderivat gemäß Anspruch 9 mit folgender Struktur:

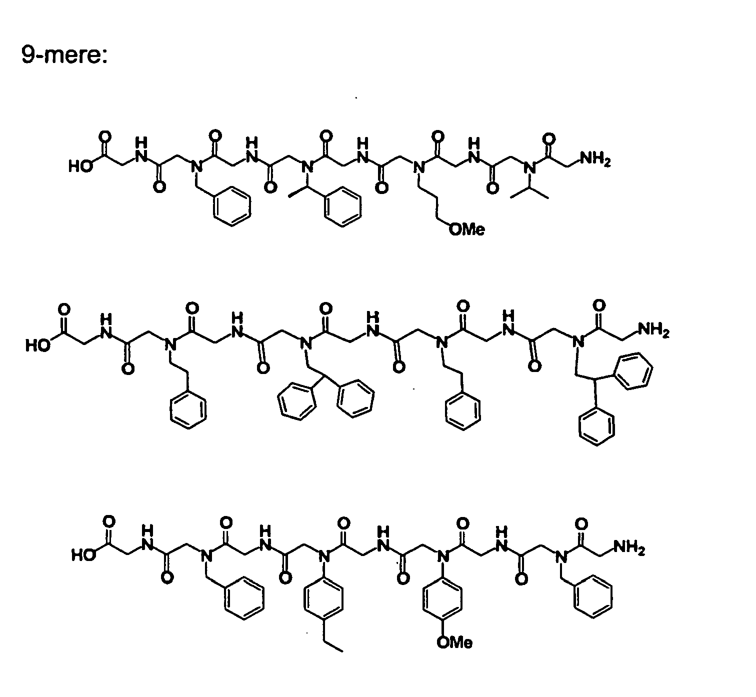

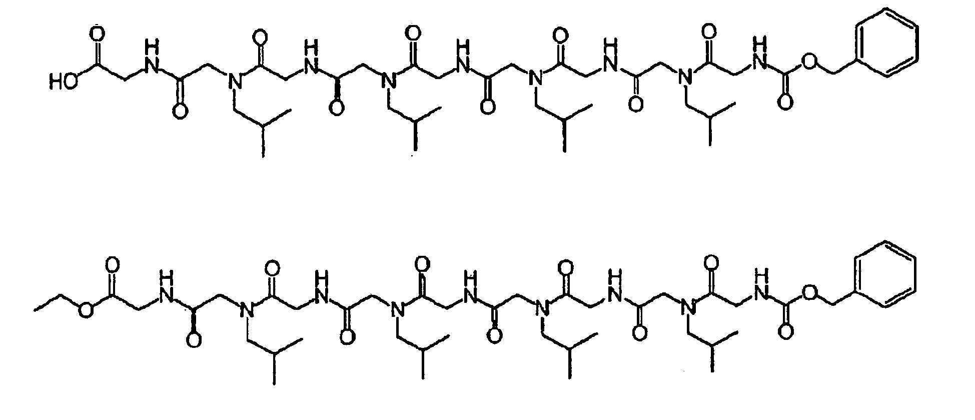

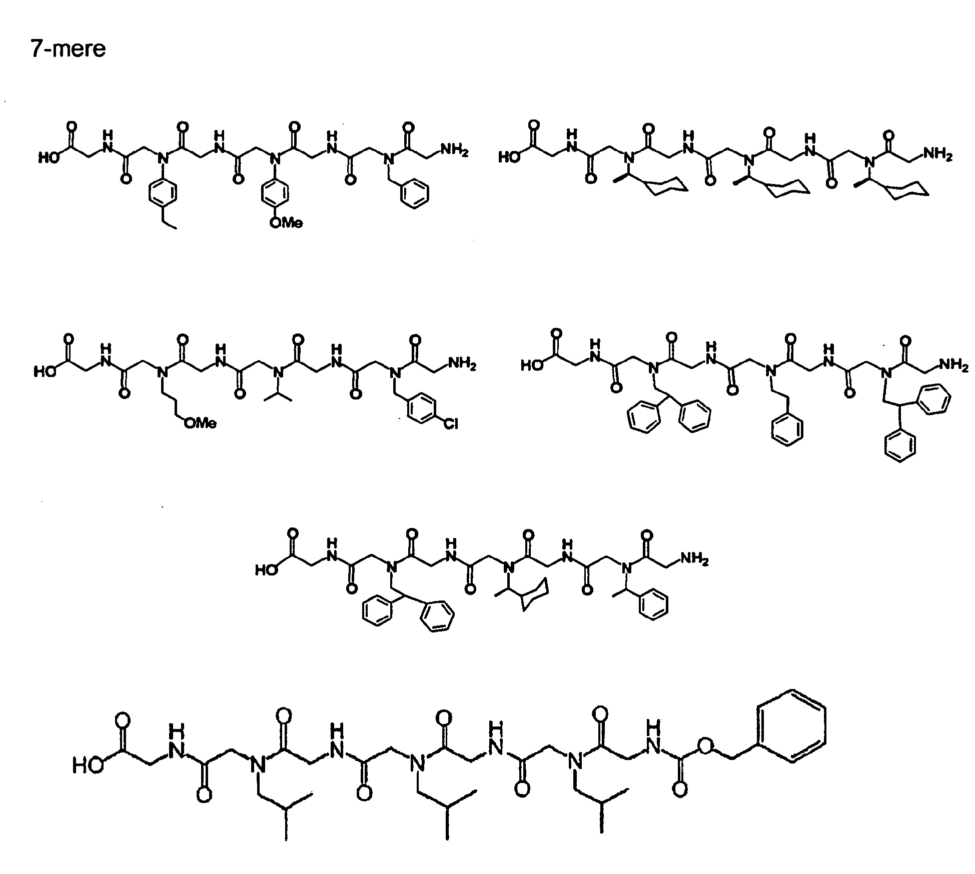

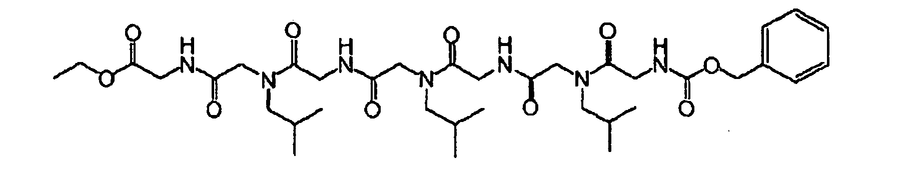

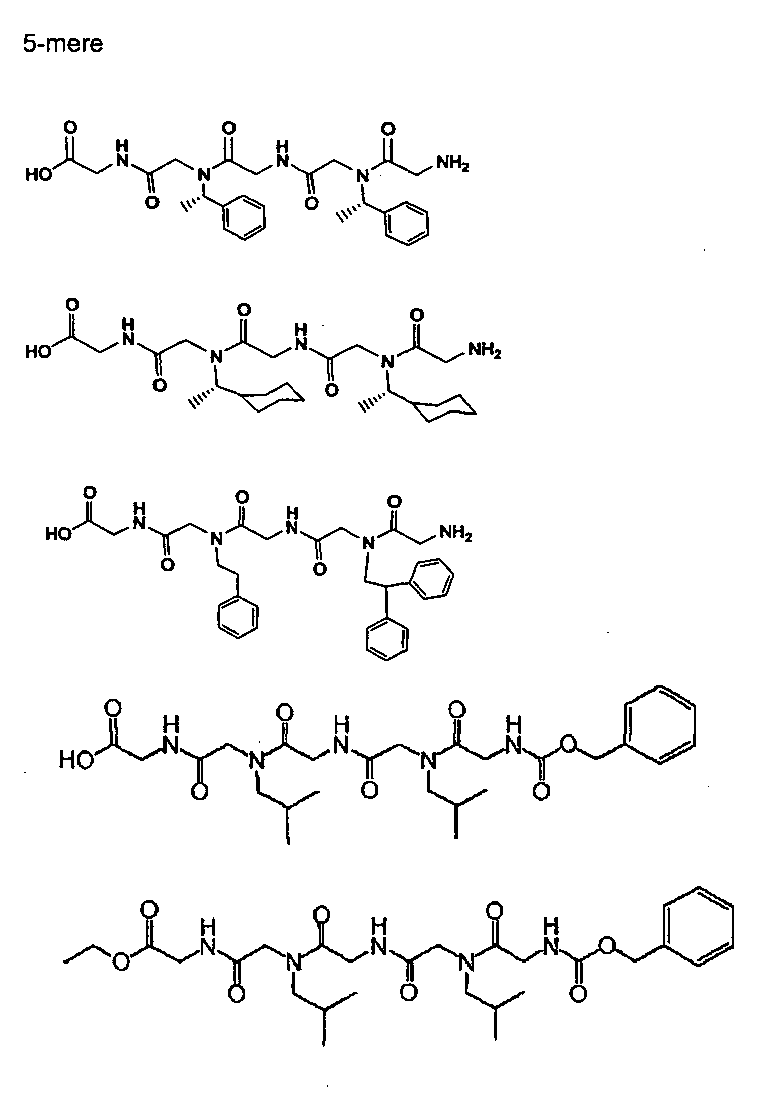

11. Peptoidderivat gemäß Anspruch 9 mit folgender Struktur: 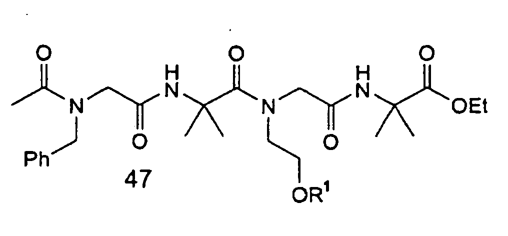 wobei R¹ wie vorstehend definiert ist.

12. Peptoidderivat gemäß Anspruch 9 mit folgender Struktur:

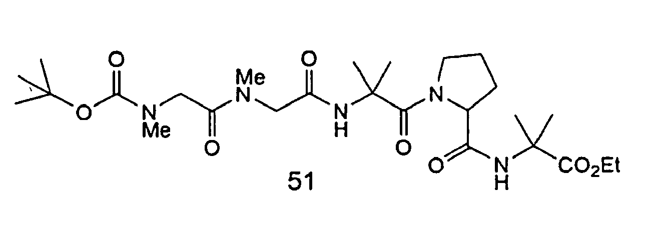

13. Peptoidderivat gemäß einem der Ansprüche 9 bis 12 in der Form eines Konjugats mit einem Wirkstoff oder einer Reportereinheit, verbunden über lipophile oder hydrophile Linkereinheiten.

14. Pharmazeutische Zusammensetzung, umfassend ein Peptoidderivat gemäß einem der Ansprüche 9 bis 13.

15. Peptoidderivats gemäß einem der Ansprüche 9 bis 13 oder pharmazeutische Zusammensetzung gemäß Anspruch 14 zur Verwendung als Antibiotika, Antiinfektiva oder für alle an die Notwendigkeit zur Zellwandpermeabilität oder Zellwandlokalisation gekoppelten pharmakologischen Anwendungen.

## Claims

1. A process for producing condensation products of N-substituted glycine derivatives (peptoids) by sequential Ugi multicomponent reactions according to scheme 1 below: 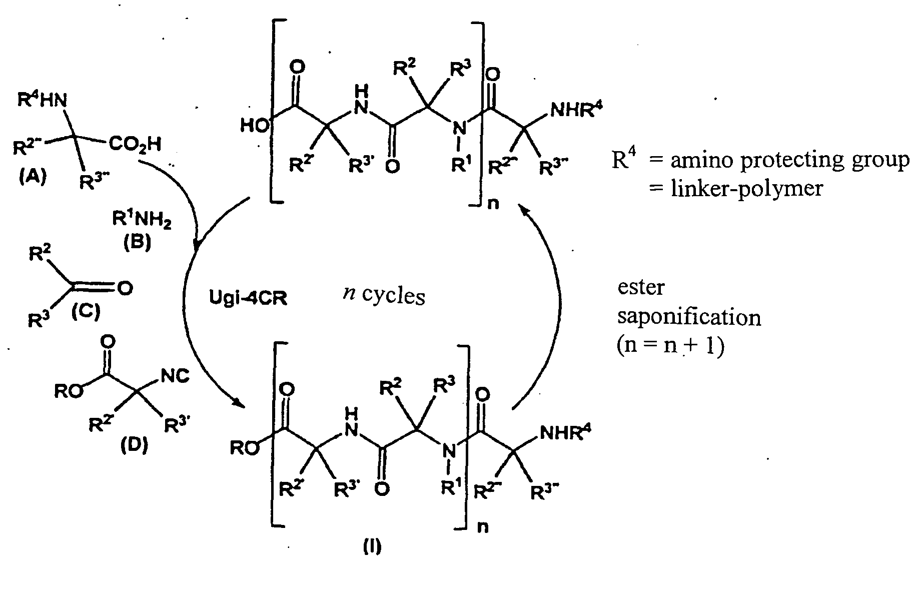 where in a first step, preferably simultaneously, an N-substituted glycine derivative (component A), a primary amine (component B), an oxo compound (component C) and an isonitrile (component D) are reacted and after ester saponification of the thus resulting product and optional removal of one or more protecting groups, the reaction is repeated n-times, wherein the product of the respective preceding step after the first step is used instead of component (A) to obtain compounds according to the following formula (I) 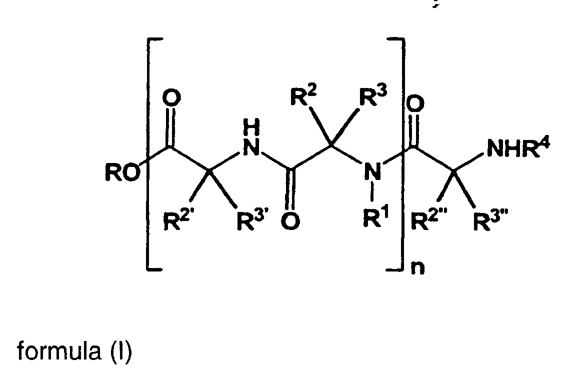 wherein
n is an integer of 2 to 100,
the residues R¹, R^{2,2',2"} and R^{3,3',3"} are, independently of each other, H, a substituted or unsubstituted alkyl, alkoxy, aryl, cycloalkyl, bicycloalkyl, tricycloalkyl, alkenyl, alkynyl, alkaryl, heteroaryl with one of more heteroatoms, selected from O, N, S, P, Si or B, or alkheteroaryl residue, with the proviso that R¹ is not H, the residue R⁴ is H or a cleavable amino protecting group, and
the residue R is H or a cleavable ester group or carboxylic acid protecting group.

2. The process according to claim 1, wherein the component (D) is an isonitrile derived from a glycine ester or amino isobutyric acid ester.

3. The process according to claim 1 or 2, wherein the component (B) is paraformaldehyde.

4. The process according to any one of claims 1 to 3, wherein as component (A), a solid-phase bonded carboxylic acid providing the initial carboxylate functionality for the Ugi multicomponent reaction, is employed.

5. The process according to any one of claims 1 to 4, wherein the residues R¹, R^{2,2',2"} and R^{3,3',3"} are, independently of each other, selected from H, straight or branched chain (C₁₋₁₂) alkyl residues, straight or branched chain (C₁₋₁₂) alkoxy residues, straight or branched chain (C₁₋₁₂) trialkylsilyl residues, (C₆₋₁₂) triaryl silyl residues, (C₃₋₈) cycloalkyl residues, (C₂₋₁₂) alkenyl residues, (C₅₋₁₂) aryl or heteroaryl residues, (C₆₋₁₂) alkaryl or alkheteroaryl residues, where the alkyl or aryl residues can each be substituted by hydroxy, alkoxy, aryloxy, alkanoyl, aroyl, carboxy, alkoxy carbonyl, amino, alkylamino, aminoalkyl, hydroxyamino, amido, carbamoyl, ureido, amidino, guanidino, cyano, azido, mercapto, alkylthio, alkylsulfoxy, alkylsulfonyl, alkylsulfenyl, aminosulfonyl, sulfhydrylalkyl, dialkenyldisulfide, fluorine, chlorine, bromine, iodine, alkyl, perfluoralkyl, heteroaryl, polyethylene glycol ethyl with a chain length of 1 to 40 and polyethylene glycolmonoether with a chain length of 1 to 40.

6. The process according to any one of claims 1 to 5, wherein the residues R are methyl or ethyl.

7. The process according to any one of claims 1 to 6, wherein the residue R⁴ is benzoyl oxycarbonyl.

8. The process according to any one of claims 1 to 7, wherein the reaction is performed in a range of -80°C - +200°C, particularly preferably in the range of 0°C - +50°C.

9. Peptoid derivatives having the structure according to the following formula (I) 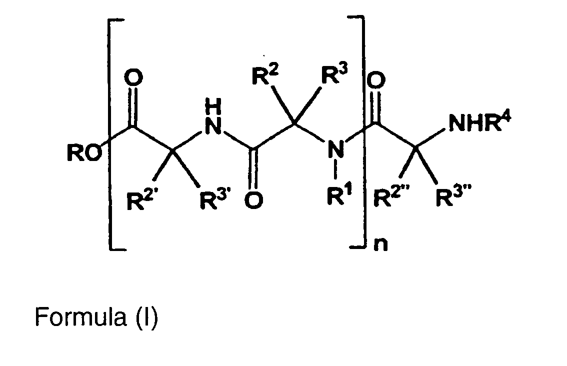 wherein
n, R¹, R^{2,2',2"}, R^{3,3',3"}, R⁴ and R are defined as given above.

10. The peptoid derivative according to claim 9, having the following structure:

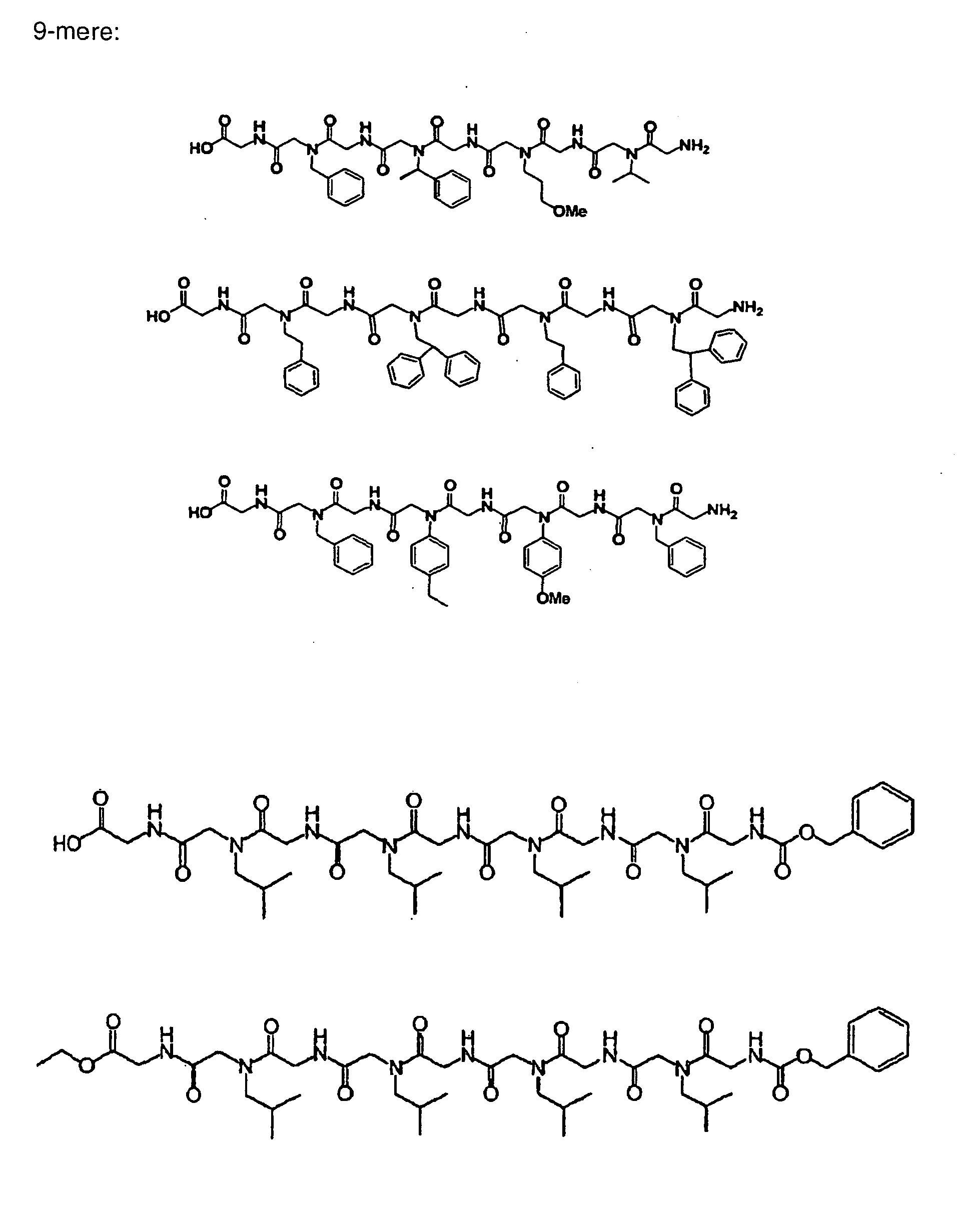

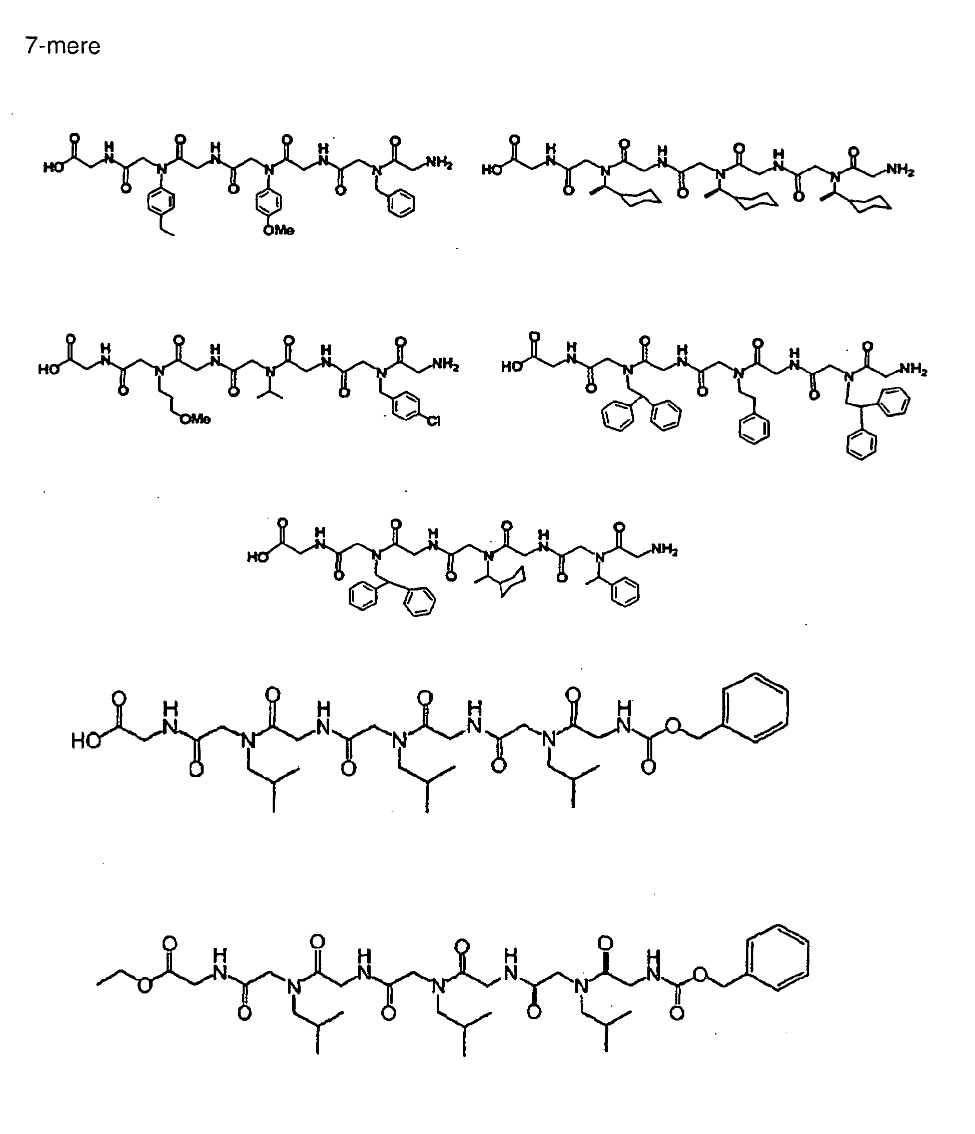

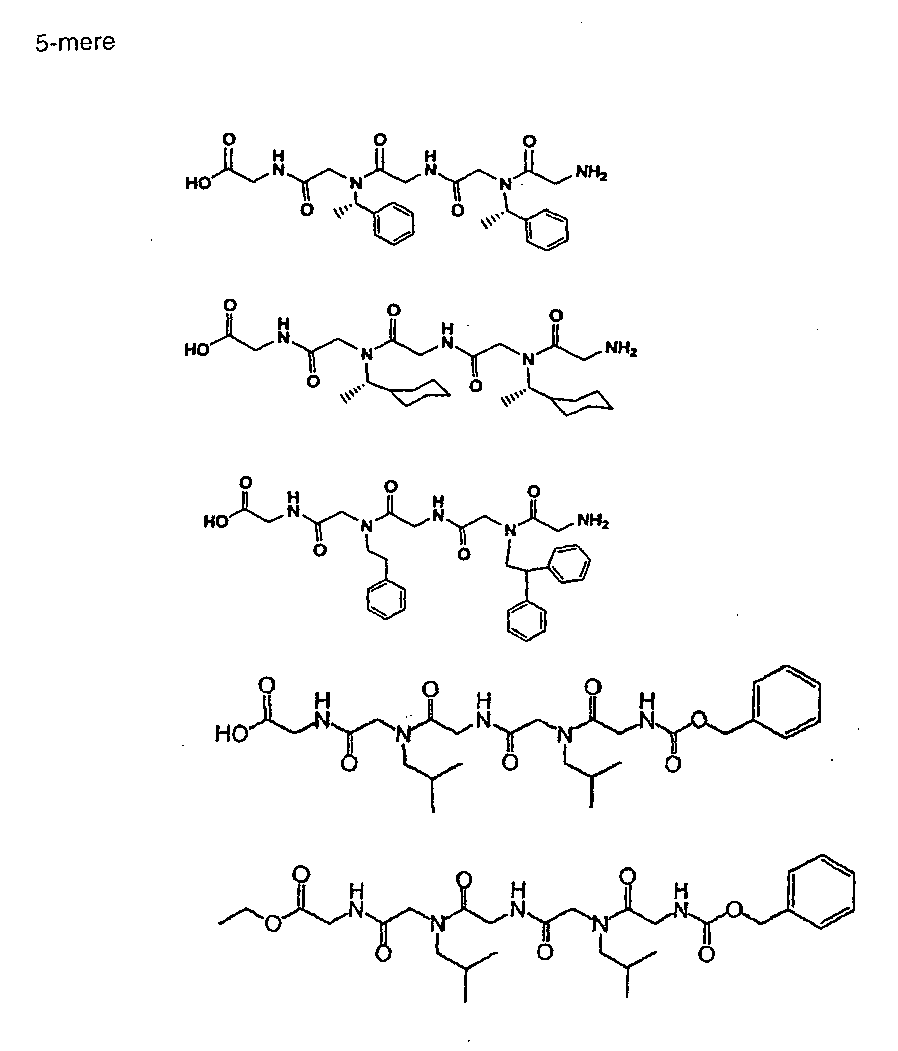

11. The peptoid derivative according to claim 9, having the following structure: 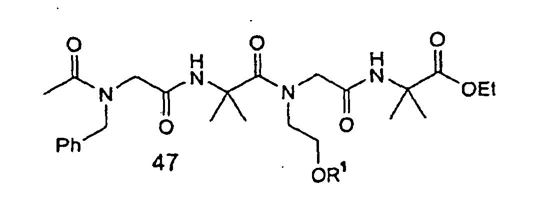 wherein R¹ is defined as given above.

12. The peptoid derivative according to claim 9, having the following structure:

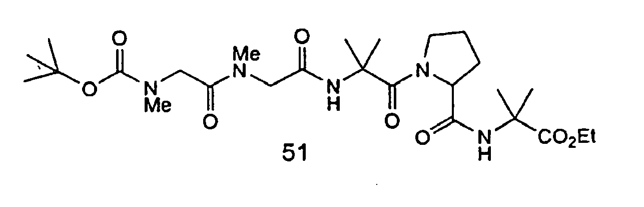

13. The peptoid derivative according to any one of claims 9 to 12, in the form of a conjugate with an active agent or a reporter moiety, connected through lipophilic or hydrophilic linker moieties.

14. A pharmaceutical composition comprising a peptoide, derivative according to any one of claims 9 to 13.

15. The peptoid derivative according to any one of claims 9 to 13 or the pharmaceutical composition according to claim 14, for use as an antibiotic agent, anti-infective agent or for all pharmacological applications connected to the necessity for cell wall permeability or cell wall localization.

## Revendications

1. Procédé de production de produits de condensation de dérivés de glycine N-substituée (peptoïdes) par des réactions séquentielles de Ugi à plusieurs composants selon le schéma 1 suivant : 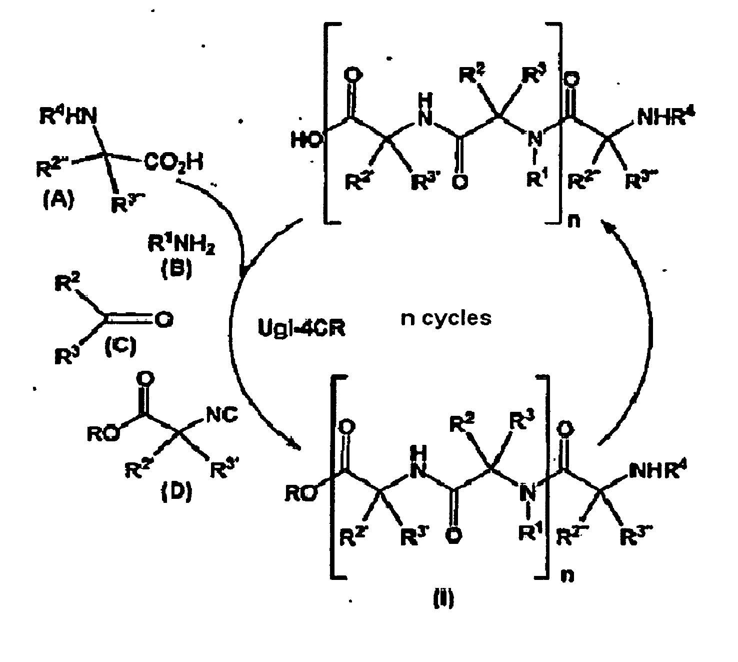
R⁴ = groupe amino protecteur
= polymère agent de liaison
Saponification des esters (n = n + 1)
dans lequel, dans une première étape, de préférence en même temps, on mélange un dérivé de glycine N-substituée (composant A), une amine primaire (composant B), un composé oxo (composant C) et un isonitrile (composant D) et après saponification des esters du produit obtenu et éventuellement élimination d'un ou plusieurs groupes protecteurs, la réaction est répétée n fois, le produit de l'étape précédente respective étant utilisé après la première étape à la place du composant (A) pour obtenir des composés selon la formule (I) suivante 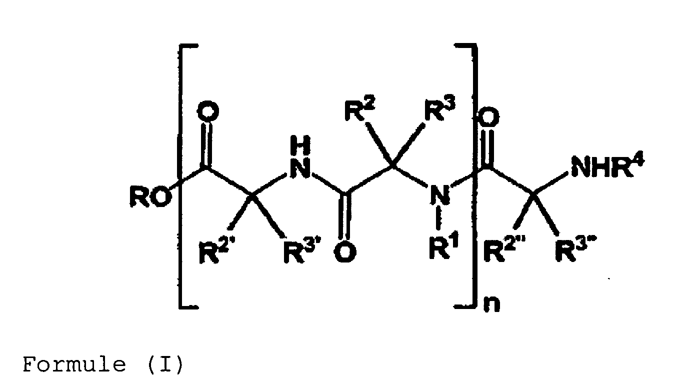 dans laquelle
n est un nombre entier de 2 à 100,
les radicaux R¹, R^{2,2',2"} et R^{3,3',3"} sont respectivement, indépendamment les uns des autres, un atome de H, un groupe alkyle, alcoxy, aryle, cycloalkyle, bicycloalkyle, tricycloalkyle, alcényle, alcinyle, alcaryle, hétéroaryle substitué ou non substitué, par un ou plusieurs hétéroatomes choisis parmi O, N, S, P, Si ou B ou un radical alc-hétéroaryle à condition que R¹ ne soit pas H,
le radical R⁴ est H ou un groupe amino protecteur pouvant être clivé et
le radical R est H ou un groupe ester pouvant être clivé ou un groupe protecteur acide carboxylique.

2. Procédé selon la revendication 1, dans lequel le composant (D) est l'isonitrile dérivé d'un ester de glycine ou d'un acide amino-isobutyrique ester.

3. Procédé selon la revendication 1 ou 2, dans lequel le composant (B) est un paraformaldéhyde.

4. Procédé selon l'une des revendications 1 à 3, dans lequel on utilise comme composant (A), un acide carboxylique lié à une phase solide qui est disponible pour la réaction de Ugi à plusieurs composants.

5. Procédé selon l'une des revendications 1 à 4, dans lequel les radicaux R¹, R^{2,2',2"} et R^{3,3',3"} sont choisis respectivement, indépendamment les uns des autres, parmi un atome de H, des radicaux alkyle (en C₁ à C₁₂) linéaires ou ramifiés, des radicaux alcoxy (en C₁ à C₁₂) linéaires ou ramifiés, des radicaux trialkylsilyle (en C₁ à C₁₂) à chaîne linéaire ou ramifiée ou des radicaux trialkylsilyle (en C₁ à C₁₂) à chaîne linéaire ou ramifiée, des radicaux triarylsilyle (en C₆ à C₁₂), des radicaux cycloalkyle (en C₃ à C₆), des radicaux alcényle (en C₂ à C₁₂), des radicaux alcinyle (en C₂ à C₁₂), des radicaux aryle ou hétéroaryle (en C₅ à C₁₂), des radicaux alcaryle ou alc-hétéroaryle (en C₆ à C₁₂), les radicaux alkyle ou aryle pouvant être substitués respectivement avec un ou plusieurs groupes hydroxy, alcoxy, aryloxy, alcanoyle, aroyle, carboxy, alcoxycarbonyle, amino, alkylamino, aminoalkyle, hydroxyamino, amido, carbamoyle, uréido, amidino, guanidino, cyano, azido, mercapto, alkylthio, alkylsulfonxy, alkylsulfonyle, alkylsulfényle, aminosulfonyle, sulfhydrylalkyle, dialcényldisulfure, fluor, chlore, brome, iode, alkyle, perfluoroalkyle, hétéroalkyle, polyéthylène-glycol éthyle présentant une longueur de chaîne de 1 à 40 et polyéthylène-glycol monoéther présentant une longueur de chaîne de 1 à 40.

6. Procédé selon l'une des revendications 1 à 5, dans lequel le radical R est un groupe méthyle ou éthyle.

7. Procédé selon l'une des revendications 1 à 6, dans lequel le radical R⁴ est un groupe benzoyloxycarbonyle.

8. Procédé selon l'une des revendications 1 à 7, dans lequel la réaction est réalisée dans une plage de -80° C à + 200° C, de manière particulièrement préférée, dans une plage de 0° C à + 50° C.

9. Dérivés de peptoïde qui présentent la structure selon la formule (I) suivante : 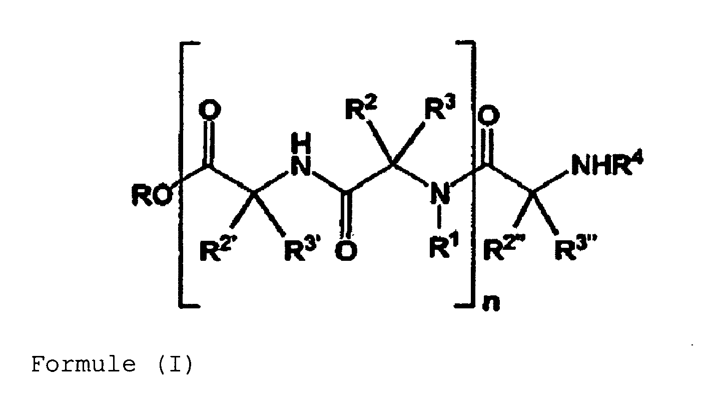 dans laquelle
n, R¹, R^{2,2',2"}, R^{3,3',3"}, R⁴ et R sont tels que définis précédemment.

10. Dérivé de peptoïde selon la revendication 9, présentant la structure suivante :

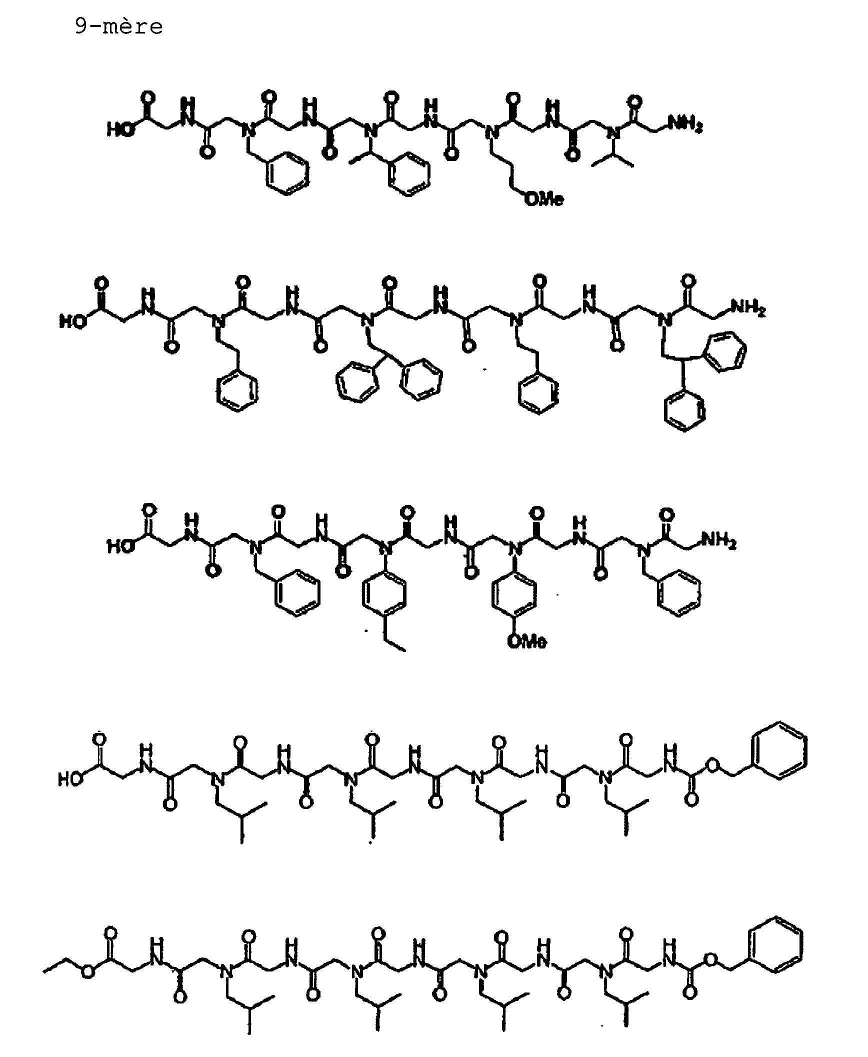

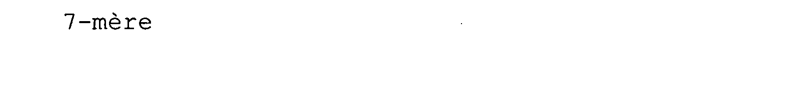

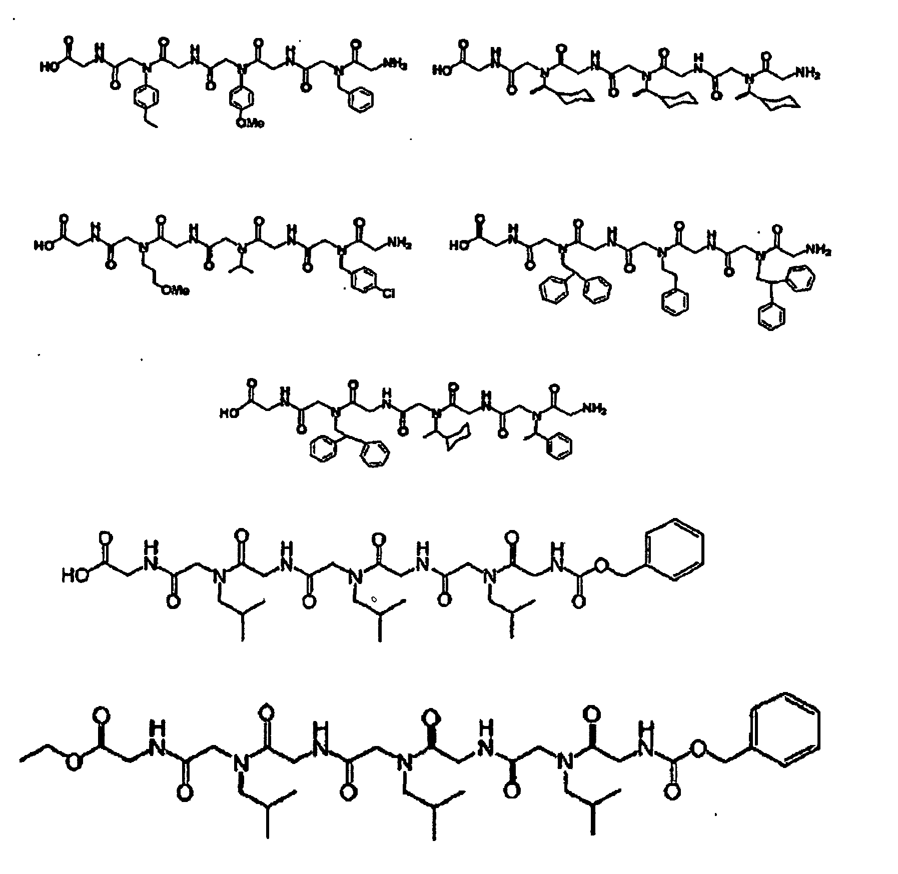

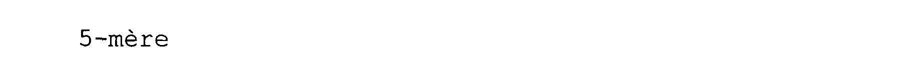

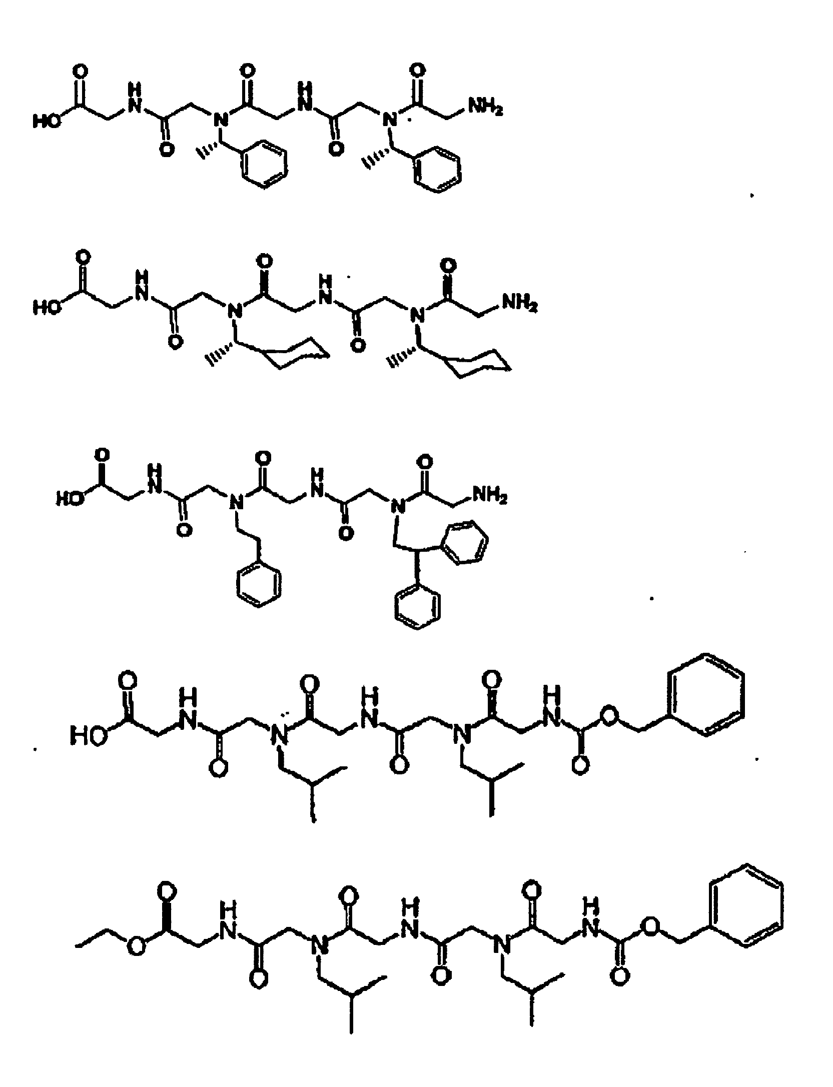

11. Dérivé de peptoïde selon la revendication 9, présentant la structure suivante : 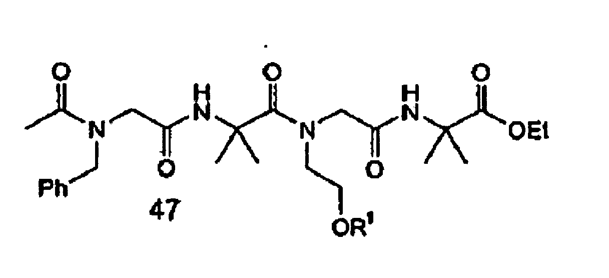 dans laquelle R¹ est tel que défini précédemment.

12. Dérivé de peptoïde selon la revendication 9, présentant la structure suivante :

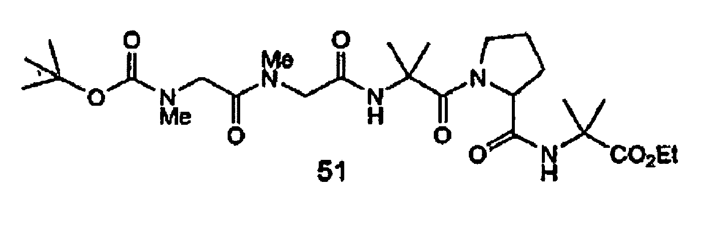

13. Dérivé de peptoïde selon l'une des revendications 9 à 12, sous la forme d'un conjugué avec un principe actif ou un motif reporter, lié par des motifs de liaison lipophiles ou hydrophiles.

14. Composition pharmaceutique, comprenant un dérivé de peptoïde selon l'une des revendications 9 à 13.

15. Dérivé de peptoïde selon l'une des revendications 9 à 13, ou composition pharmaceutique selon la revendication 14 pour l'utilisation comme antibiotique, anti-infectieux ou pour toutes les utilisations pharmacologiques associées à la nécessité d'obtenir une perméabilité de la paroi cellulaire ou la nécessité de localisation de la paroi cellulaire.
